(19) Europäisches Patentamt
European Patent Office
Office européen des brevets

(11) Publication number: 0 167 943 A2

# (12) EUROPEAN PATENT APPLICATION

(21) Application number: 85108034.1

(22) Date of filing: 28.06.85

(51) Int. Cl.[4]: C 07 D 401/12
A 61 K 31/44

(30) Priority: 05.07.84 GB 8417171

(43) Date of publication of application:
15.01.86 Bulletin 86/3

(84) Designated Contracting States:
BE CH DE FR GB IT LI NL

(71) Applicant: BEECHAM GROUP PLC
Beecham House Great West Road
Brentford Middlesex TW8 9BD(GB)

(72) Inventor: King, Francis David
8 Heath Row Bishop's Stortford
Hertfordshire(GB)

(72) Inventor: Burrell, Gordon
25 Church Leys Harlow
Essex CM18 6BY(GB)

(72) Inventor: Baker, Karen
16 The Maples Harlow
Essex CM19 4QY(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

(54) **Benzimidazole derivatives, process for their preparation, and their use as pharmaceuticals.**

(57) Compounds of formula (I):

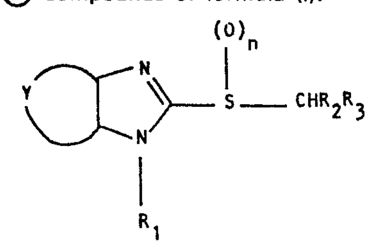

(I)

or a pharmaceutically acceptable salt, a quaternised derivative or a pharmaceutically acceptable solvent thereof; wherein

Y forms an optionally substitute phenyl ring;

n is zero or one;

$R_1$ is H, $C_{1-6}$ alkanoyl, $C_{1-6}$ alkanesulphonyl, or optionally substituted arylsulphonyl, aryl $C_{1-6}$ alkanoyl or aryl $C_{1-4}$ alkyl;

R is hydrogen or $C_{1-4}$ alkyl; and

$R_3$ is pyridyl group substituted by at least one group selected from $OR_4$ or $O(CH_2)_mOR_4$ wherein $R_4$ is an optionally substituted aryl or aralkyl group of up to 10 carbon atoms and m is an integer of from 1 to 4; and by up to three further substituents one of which may be joined to $R_2$ to form a carbocylic ring of up to 7 ring atoms having gastric secretion inhibiting activity, a process for their preparation and their use as pharmaceuticals.

EP 0 167 943 A2

B1678

TITLE MODIFIED
see front page

NOVEL COMPOUNDS

This invention relates to novel compounds, to pharmaceutical compositions containing them, to a process for their preparation and to their use.

The compounds of the invention inhibit gastric secretion and inhibit the enzyme $(H^{+}+K^{+})$-ATPase, and thus may be used in the treatment of disorders caused or exacerbated by excess gastric acid secretion such as peptic ulcer and Zollinger-Ellison syndrome.

European Patent Application Nos.007434 and 0005129 disclose various alkoxy substituted pyridylthiobenzimidazoles and alkoxy substituted pyridylsulphinylbenzimidazoles having gastric acid secretion inhibiting activity.

Accordingly, the present invention provides a compound of formula (I):

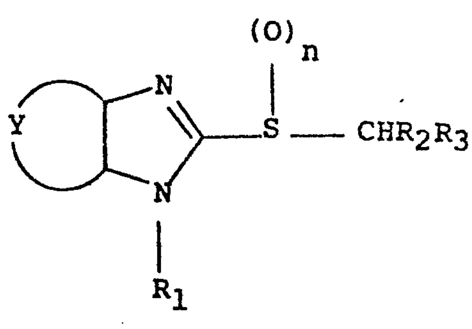

(I)

or a pharmaceutically acceptable salt, a quaternised derivative or a pharmaceutically acceptable solvent thereof;

wherein:

Y forms an optionally substituted phenyl ring;

n is zero or one;

$R_1$ is H, $C_{1-6}$ alkanoyl, $C_{1-6}$ alkanesulphonyl, or optionally substituted arylsulphonyl, aryl $C_{1-6}$ alkanoyl or aryl $C_{1-4}$ alkyl;

$R_2$ is hydrogen or $C_{1-4}$ alkyl; and

$R_3$ is pyridyl group substituted by at least one group selected from $OR_4$ or $O(CH_2)_mOR_4$ wherein $R_4$ is an optionally substituted aryl or aralkyl group of up to 10 carbon atoms and m is an integer of from 1 to 4; and by up to three further substituents one of which may be joined to $R_2$ to form a carbocylic ring of up to 7 ring atoms.

Favourably $R_3$ is an optionally substituted 2-pyridyl group.

Suitable further substituents for $R_3$ include halogen, $C_{1-6}$ alkyl, $C_{1-6}$ alkoxy, $C_{1-6}$ alkylthio, $C_{1-6}$ alkoxy $C_{1-6}$ alkoxy, carboxy esterified carboxy, or amino optionally N-substituted by one or two groups independently selected from $C_{1-6}$ alkyl, phenyl or phenyl $C_{1-4}$ alkyl or optionally N.N-disubstituted by $C_{4-5}$ polymethylene or $C_{3-4}$ polymethylenecarbonyl.

As used herein the term 'aralkyl' includes aryl $C_{1-4}$ alkyl such as phenyl $C_{1-4}$ alkyl and naphthyl $C_{1-4}$ alkyl and heteroaryl $C_{1-4}$ alkyl such as furyl $C_{1-4}$ alkyl.

As used herein the term 'aryl' includes phenyl and naphthyl. Suitable example of an arylsulphonyl group for $R_1$ is benzenesulphonyl and a suitable aryl $C_{1-6}$ alkanoyl group is benzoyl.

Any aryl groups may be optionally substituted by one or two substituents selected from halogen, $C_{1-6}$ alkyl, $C_{1-6}$ alkoxy and $CF_3$.

Any optional substituents for $R_1$ are usually present on the aryl ring.These optional substituents suitably consist of one or two members selected from the group consisting of $C_{1-4}$ alkyl optionally substituted by halogen, such as trifluoromethyl, $C_{1-4}$ alkoxy, halogen,nitro, $C_{1-6}$ alkoxycarbonyl and carboxyl.

Usually $R_4$ is substituted in the aryl ring portion thereof. Suitable optional substituents are one or two members selected from the group consisting of halo, $C_{1-6}$ alkyl, $C_{1-6}$ alkoxy, $C_{1-6}$ alkylthio, cyano or nitro. A particularly preferred $R_4$ is phenyl para-substituted by halo, such as chloro or fluoro.

Favourably the group Y includes one or two substituents selected from halo, $C_{1-6}$ alkyl, halo substituted $C_{1-6}$ alkyl, $C_{1-6}$ alkoxy, $C_{1-6}$ alkylthio, $C_{1-10}$ carboxylic acyl, (such as $C_{1-7}$ alkanoyl and $C_{3-8}$ alkyl carbonyl), $C_{1-7}$ carboxylic acylamino, carboxy, $C_{1-6}$ alkoxycarbonyl, $C_{1-6}$ alkylsulphonylamino, N-($C_{1-6}$ alkylsulphonyl)-$C_{1-4}$ alkylamino, cyano, nitro, or amino, amido or sulphonylamino any of which is optionally N-substituted by one or two groups selected from $C_{1-6}$ alkyl or phenyl or optionally N-disubstituted by $C_{4-5}$ polymethylene; or phenyl optionally substituted by one or two substituents independently selected from halo, $C_{1-6}$ alkyl, $C_{1-6}$ alkoxy, $C_{1-6}$ alkylthio, $C_{1-7}$ carboxylic acyl, $C_{1-7}$ carboxylic acylamino, $C_{1-6}$ alkylsulphonylamino, N-($C_{1-6}$ alkylsulphonyl)-$C_{1-4}$ alkylamino, cyano, or nitro, amino optionally N-substituted by one or two groups selected from $C_{1-6}$ alkyl or phenyl or optionally N-disubstituted by $C_{4-5}$ polymethylene, or carboxy or $C_{1-6}$ alkoxycarbonyl.

A group of compounds within formula (I) is of formula (II):

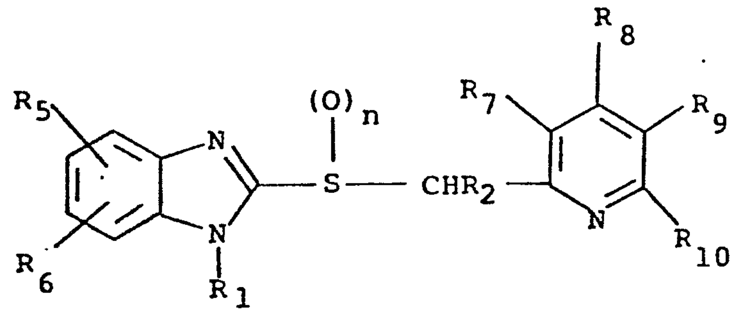

(II)

or a pharmaceutically acceptable salt, a quaternised derivative or a pharmaceutically acceptable solvate thereof;

wherein $R_1$, $R_2$ and n are as hereinbefore defined in relation to formula (I);

$R_5$ and $R_6$ are independently hydrogen or a group selected from the substituents for Y defined above;

$R_8$ is a group selected from $OR_4$ or $-O(CH_2)m-O-R_4$;

$R_9$ and $R_{10}$ are independently selected from hydrogen, $-OR_4$, $-O(CH_2)_mR_4$, halogen, $C_{1-6}$ alkyl, $C_{1-6}$ alkoxy, $C_{1-6}$ alkylthio, $C_{1-6}$ alkoxy $C_{1-6}$ alkoxy, carboxy, esterified carboxy, or amino optionally substituted by one or two groups independently selected from $C_{1-6}$alkyl-phenyl, or phenyl-$C_{1-4}$ alkyl or optionally N,N-disubstituted by $C_{4-5}$ polymethylene or $C_{3-4}$ polymethylenecarbonyl; and $R_7$ is a group defined above for $R_{10}$ and $R_9$ or together with $R_2$ form $C_{2-9}$ alkylene; wherein $R_4$ and m are as hereinbefore defined in relation to formula (I).

Examples of $R_5$ and $R_6$ include hydrogen, halo, such as chloro or bromo, methyl, trifluoromethyl, amino or methoxy.

$R_1$ is preferably hydrogen.

Examples of $R_2$ include hydrogen.

Examples of $R_7$, $R_9$ and $R_{10}$ include hydrogen and $C_{1-4}$ alkyl such as methyl.

Examples of $R_8$ include phenyl $C_{1-4}$ alkoxy, optionally substituted as hereinbefore defined, in particular benzyloxy.

In one particular embodiment $R_7$ is methyl, $R_8$ is benzyloxy or 4-fluorobenzyloxy and $R_9$ and $R_{10}$ are both hydrogen.

It will be appreciated that the compounds of formulae (I) or(II) may be capable of existing in more than one tautomeric form when $R_1$ = H. The present invention extends to each of these forms and to mixtures thereof.

It will of course be realised that the compounds of the formulae (I) or (II) may have chiral centres, and thus be capable of existing in a number of stereoisomeric forms. The invention extends to each of these stereoisomeric forms (including enantiomers) and to mixtures thereof (including racemates).

An example of a chiral centre is the carbon atom of the moiety $-CHR_2-$ when $R_2$ is other than hydrogen.

Pharmaceutically acceptable salts of the compounds of the formulae (I) and (II) include pharmaceutically acceptable inorganic salts such as sulphate, nitrate, phosphate, borate, hydrochloride and hydrobromide, and pharmaceutically acceptable organic acid additions salts such as acetate, fumarate, tartrate, citrate, lactate, salicylate, maleate, succinate, benzoate, ascorbate, methanesulphonate, mandelate, α-ketoglutarate, α-glycerophosphate, and

the acid addition salt is the hydrochloride salt.

Examples of quaternised derivatives include compounds of the formula (I) and (II) quaternised by $R_{18}Q$, where $R_{18}$ is $C_{1-4}$ alkyl, $C_{3-6}$ cycloalkyl, $C_{3-6}$ cycloalkyl, $C_{1-4}$ alkyl or phenyl $C_{1-4}$ alkyl and Q is halide such as chloride, bromide or iodide.

A particular compound of the formulae (I) or (II) or its acid addition salt can form salts with alkali and alkaline earth metals, usually sodium and potassium, and ammonium and substituted ammonium salts.

Compounds of formula (I) or (II) and their pharmaceutically acceptable salts or quaternised derivatives may form solvates with pharmaceutically acceptable solvents and the invention extends to such solvates.

Crystalline compounds and salts are favoured.

The invention also provides a process for the preparation of a compound of the formula (I) or a pharmaceutically acceptable salt, a quaternised derivative or a pharmaceutically acceptable solvate thereof, which process comprises reacting a compound of formula (III) or an acid addition salt thereof:

$$R_3 - R_{11} \qquad (III)$$

wherein $R_3$ is as hereinbefore defined in relation to formula (I), with a compound of formula (IV):

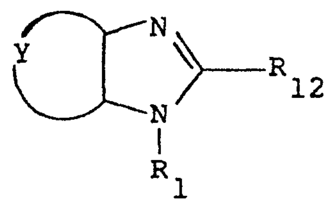

(IV)

wherein $R_1$ and Y are as hereinbefore defined in relation to formula (I); and

a) $R_{11}$ is a group displaceable by a nucleophile; and $R_{12}$ is $CH_3SO$;

b) $R_{11}$ is $CHR_2Q_1$ where $R_2$ is as hereinbefore defined, and $Q_1$ is a group displaceable by a nucleophile; and $R_{12}$ is HS; or

c) $R_{11}$ is $CHR_2SH$ where $R_2$ is as hereinbefore defined; and $R_{12}$ is a group displaceable by a nucleophile;

thereafter if desired carrying out one or more of the following steps;

(i) oxidising a compound of formula (I) wherein n is zero to a compound of formula (I) wherein n is one;

(ii) converting any variable group to another corresponding variable R group; and

(iii) salifying or quaternising the resulting compound of the formula (I).

Suitable examples of $R_{11}$ in variant a) above include halide such as Cl or Br.

Suitable examples of $Q_1$ and $R_{12}$ in process variants b) and c) respectively include halide such as Cl, Br or I, and labile acyloxy such as $OSO_2CH_3$ or $OSO_2p\text{-}C_6H_4CH_3$ (mesyloxy and tosyloxy).

Reaction in all variants is generally effected at a non-extreme temperature, such as a moderately elevated temperature for example solvent reflux temperature, such as 50 to 150°C, for example 75 to 100°C, in an inert solvent, preferably in the presence of an acid acceptor. The acceptor is suitably an inorganic acid acceptor, such as a strong base for example sodium hydride, butyl lithium or lithium diisopropylamide; a moderately strong base, for example sodium hydroxide; or a moderate base for example calcium carbonate, sodium carbonate or potassium carbonate. In some cases the moderate base acid acceptor is favourably an organic base such as a tertiary amine, e.g. triethylamine, trimethylamine, pyridine or picoline. The most suitable acceptor depends on the particular variant.

Thus for example, for variant a) a strong base is appropriate. For variant b) or c) a moderately strong base or moderate base is appropriate.

The inert solvent can be any solvent inert to both reactants and appropriate to the leaving group, the acid acceptor and desired reaction remperature. Suitable solvents include lower alkanols such as ethanol, dioxan, dimethylformamide (DMF) toluene, diethyl ether,or methylene chloride.

For process variant b) in particular suitable solvents include polar solvents such as DMF or ethanol. Where reaction is effected in the presence of a base which is insoluble in the polar solvent, water may be added to the solvent. Reaction is generally effected at moderately elevated temperatures as mentioned hereinbefore, such as reaction mixture reflux temperature or at about 100°C, if lower.

It will be appreciated that when the compound of formula (III) or (IV) contains an unsubstituted amino group, such a group will generally be protected, during at least the main reaction of the invention by a conventional N-protecting group for examples an acyl group such as acetyl or phthalyl. Protection may be effected by reaction with an acylating agent such as the relevant acyl chloride or anhydride. Deprotection may be effected for example by base hydrolysis of an acetyl protecting group or treatment of a phthalimide protecting group with hydrazine hydrate in a lower alkanol.

Process variants b) and c) produce compounds of formula (I) wherein n is O. If a compound of formula (I) is desired wherein n is, it is necessary to oxidise the compound resulting from the process.

Subsequent oxidation may be carried out at below ambient temperatures in a non-aqueous solvent, such as a chlorinated hydrocarbon, in the presence of an organic peracid, such as 3-chloroperbenzoic acid, or in water in the presence of a soluble strong inorganic oxidant, such as aqueous hydrogen peroxide. It will be realised that this process may also N-oxidise any tertiary amine moiety and suitable precautions will

routinely be taken by the skilled man, if it is desired to avoid this.

The conversions in step (iii) above are carried out by conventional methods.

Examples of such conversions include the conversion of Y group substitutent to another. In particular

a) hydrogen is convertible to a nitro substitutent by nitration;

b) a nitro substituent is convertible to an amino substituent by reduction;

c) an acylamino substituent is convertible to an amino substituent by deacylation;

d) an amino substituent is convertible to acylamino substituent by acylation;

e) hydrogen is convertible to a halo substituent by halogenation;

f) a fluoro or chloro substituent is convertible to an optionally substituted amino substituent by reaction with suitable amine or ammonia;

g) an amino substituent is convertible to a halo, cyano, or hydrogen substituent by diazotisation and simultaneous nitrogen elimination with halogenation, cyanation, or reduction;

h) an amino substituent is convertible to an alkylated amino substituent by alkylation or reductive alkylation; and

i) a carboxy substituent is convertible to a $C_{1-6}$ alkoxycarbonyl substituent by esterification.

In regard to (a), nitration is carried out in accordance with known procedures.

In regard to (b), the reduction is carried out using methods conventional for reducing nitro groups on aromatic nuclei, for example using Raney nickel, stannous chloride or iron powder in glacial acetic acid or hydrochloric acid.

In regard to (c), deacylation is carried out by treatment with a base, such as an alkali metal hydroxide.

In regard to (d), the acylation is carried out with an acylating agent, such as the corresponding acid anhydride or acid chloride. Formylation is carried out with formic acid.

In regard to (e), halogention is carried out with conventional halogenating agents, under conventional reaction conditions for the halogenation of aromatic nuclei, that is with the relevant halogen in the presence of a Lewis acid catalyst, such as ferric chloride, zinc chloride or boron trifluoride, in an inert organic sovlent such as chloroform or dichloromethane, at temperatures below ambient. Alternatively a polar solvent, such as glacial acetic acid without a catalyst may be used.

In regard to (f), the amination is carried out under conventional conditions using an inert solvent such as ethanol or an excess of amine also functioning as the solvent.

(g) may be effected by reacting an alkali metal nitrite, a strong inorganic acid and the reaction product in aqueous solution at 10 to -10$^{o}$C.

Subsequent simultaneous nitrogen elimination and halogenation, cyanation or reduction may be effected by treating the diazotisation product with a halide, cyanide or hydride source, under either Sandmeyer or Schiemann reaction conditions for halide and nitrile, or by treatment with hypophosphorous acid for hydride respectively.

(h) and (i) may be effected entirely conventionally.

For $R_3$;

j) H may be converted to acyl by conventional acylation.

k) any of the acyl groups listed hereinbefore for $R_{11}$ may be converted to hydrogen by conventional deacylation.

l) carboxy may be converted to $C_{1-6}$ alkoxycarbonyl by conventional esterification.

In regard to (j) O-acylation is carried out under conventional conditions with an acylating agent which has an acyl group capable of forming an hydrolysable acyloxy group and a leaving group, such as halide, for example chloride and bromide, and hydrogen. When halide is the leaving group, the reaction is generally carried out in the presence of a base. When hydroxy is the leaving group, the reaction is generally carried out in the presence of a dehydrating agent, at a non-extreme temperature, such as ambient temperature.

In regard to (k), deacylation is carried out by treatment with a base, such as an alkali metal hydroxide.

In regard to (l) esterification is entirely conventional.

Conversions (a) to (l) are only exemplary and are not exhaustive of the possibilities.

In general, interconversion of $R_7$,$R_8$,$R_9$ and $R_{10}$ to other $R_7$,$R_8$,$R_9$ and $R_{10}$ will not be readily effected. However, such interconversions as are possible, and suitable methods and conditions for effecting them, will be readily apparent to the skilled man.

The choice or necessity of variable interconversion will be dictated by the nature and position of the variable, as will the choice of intermediate or compound in which interconversion is effected.

In all the foregoing interconversions, the effect, if any, on other substituents should be considered, and such reagents as are appropriate should be selected together with the adoption of such routine precautionary measures as are necessary.

It is however preferred that any conversions are carried out at the earliest stage possible in the synthesis.

The invention also provides a process, for the preparation of a compound of formula (I) as hereinbefore defined or a pharmaceutically acceptable

salt or quaternary derivative thereof, which process comprises reacting a compound of formula (V) or a pharmaceutically acceptable salt thereof:

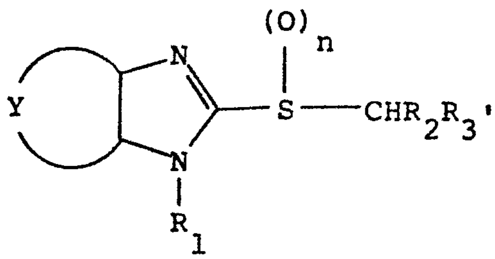

(V)

wherein

$R_1$, $R_2$,Y and n are as defined in relation to formula (I) and $R_3'$ is a pyridyl group substituted by at least one hydroxy or $C_{1-4}$alkoxy groups and up to three further substituents independently selected from halogen, $C_{1-6}$ alkyl, $C_{1-6}$ alkoxy, $C_{1-6}$ alkylthio, $C_{1-6}$-alkoxy $C_{1-6}$ alkoxy, carboxy esterfied carboxy, or amino optionally N-substituted by one or two groups independently selected from $C_{1-6}$ alkyl, phenyl or phenyl $C_{1-4}$ alkyl or optionally N.N-disubstituted by $C_{4-5}$ polymethylene or $C_{3-4}$ polymethylenecarbonyl, or one of said further substituents is joined to $R^2$ to form a carbocylic ring of up to 7 ring atoms; with a compound of formula $R_{13}Q_2$;

wherein:

$R_{13}$ is $R_4$ as hereinbefore defined or when $R_3'$is substituted by hydroxy, $R_{13}$ can be $(CH_2)mOR_4$ and $Q_2$ is a leaving group; in which m is as hereinbefore defined; and thereafter if necessary, carrying out one or more of the following steps:

(i) oxidising a compound of formula (I) wherein n is zero to a compound of formula (I) wherein n is one;

(ii) converting any variable R group to another corresponding variable R group; and

(iii) salifying or quaternising the resulting compound of the formula (I).

Suitable examples of $Q_2$ include halide such as Chloro or bromo.

Process conditions may be conventional conditions for the alkylation of an aromatic hydroxy substituent.

The interconversion of variables groups may be effected as described hereinbefore.

In the above described processes, compounds of the formula (I) may be salified in entirely conventional manner by reacting a compound of the formula (I) in base form with a chosen acid to form acid addition salts.

The quaternary derivatives of the compounds of the formula (I) may be prepared in conventional manner, such as by reaction of the chosen compound of the formula (I) with a compound $R_{18}Q$ as defined. This reaction is suitably carried out in an appropriate solvent such as acetone, methanol or ethanol.

Salts of compounds of the formula (I) containing a carboxy groups may be formed conventionally by reacting a compound of the formula (I) with a corresponding base, for example an alkali metal hydroxide, an alkaline earth metal hydroxide or an optionally substituted ammonium hydroxide.

The preparation of intermediates for the preparative process of the invention may be effected by building up the intermediates in any given process or process variant by processes analogous to those in other relevant variants of the first process of the invention or by conventional oxidation as described hereinbefore for a compound of the formula (I), or such intermediates are known compounds or are preparable analogously to or routinely derivable from known compounds.

The intermediates of formula (III) in all the variants of the first process are known compounds, or are preparable analogously to, or are routinely derivable from known compounds.

For example the intermediates of formula (III) in variant a) of the first process of the invention, i.e. wherein $R_{10}$ is a group displaceable by a nucleophile, in particular chloro or bromo, may be prepared by halogenation of the corresponding 2-pyridone. Suitable halogenating agents include phosphorus oxychloride, phosphorus oxybromide, thionyl chloride, and phosphorus pentachloride used under conventional conditions for each given reagent.

It is believed that the intermediates of formula (IV) in all the variants of the first process are known compounds or are preparable analogously to, or are routinely derivable from known compounds.

However, by way of example the preparation of various intermediates of formula (IV) are described hereinafter:

In variant (a)of the first process, the intermediates of formula (IV), i.e. wherein $R_{11}$ is $CH_3SO$ may be prepared by oxidising in the manner described hereinbefore for a compound of formula (I), a corresponding compound wherein the $CH_3SO$ group is replaced by $CH_3S$. This compound may be prepared by conventional S-alkylation of a compound of formula (V) wherein $R_{11}$ is SH, i.e. the intermediate of process variant (b).

In process variant (b), the intermediate of formula (IV), i.e. wherein $R_{11}$ is HS may be prepared by reacting a compound of formula (V) wherein $R_{11}$ is a group displaceable by a nucleophile with thiourea followed by base hydrolysis of the thiuronium salt.

The intermediates of formula (V) are believed to be novel and as such form an aspect of the present invention.

They are preparable by a process analogous to the first process of this invention. The skilled man will appreciate that in such a process it is desirable to protect any $R_7$ hydroxy group to prevent electrophilic attack on the group oxygen atom. Protection may be achieved for example using a $C_{1-6}$ alkyl group, which may be conventionally converted to hydrogen subsequent to the main reaction, for example using warm hydrobromic acid or iodotrimethylsilane.

The compounds of formulae (I)and (II), pharmaeutically acceptable salts and quaternised derivates thereof, and pharmaceutically acceptable solvates of any of the foregoing may be used in the treatment of disorders caused or exacerbated by excess gastric acid secretion such as peptic ulcer and Zollinger-Ellison syndrome.

The invention thus also provides a pharmaceutical composition comprising a compound of the invention, in particular a compound of formula (I), a pharmaceutically acceptable salt, quaternised derivative or a pharmaceutically acceptable solvate thereof, together with a pharmaceutically acceptable carrier.

The compositions may be formulated for administration by any route, although oral administration is preferred. The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling, or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel,hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in

the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a sufactant or wetting agent is included in the composition to facilitate uniform distribution of the compound.

When appropriate the compositions of this invention may be presented as an aerosol for oral administration, as a microfine powder for insufflation, or as a suppository for rectal or vaginal administration. Suitable unit dose forms include tablets, capsules and powders in sachets or vials, and preferred forms include shaped oral unit doses, such as tablets and capsules.

The compositions may contain from 0.1% to 99% by weight, preferably from 10-60% by weight, of the active meterial, depending on the method of administration.

The invention also provides a method of treatment or prophylaxis of disorders, such as peptic ulcers, in mammals including humans, caused or exacerbated by excess gastric acid secretion, which comprises the administration of an effective amount of a compound of formula (I), or a pharmaceutically acceptable salt or quaternised derivative thereof, or a pharmaceutically acceptable solvate of any of the foregoing, to the sufferer.

An amount effective to treat the disorders hereinbefore described depends on the relative efficacies of the compounds of the invention, the nature and severity of the disorder being treated and the weight of the mammal. However, a unit dose will normally contain 1 to 2000 for example 5 to 1000 mg of the compound of the invention. Unit doses will normally be administered at least once a day, for example 1,2,3,4,5 or 6 times a day such that the total daily dose is normally in the range 0.1 to 30 mg/kg per day, i.e. 7 to 2000 mg/day for a 70kg human adult.

As is common practice, the compositions will usually be accompanied by written or printed directions for use in the medical treatment concerned.

The invention also provides a compound of formula (I) or a pharmaceutically acceptable salt, a quaternised derivative or a pharmaceutically acceptable solvate thereof for use in therapy, in particular for use in the treatment or prophylaxis of disorders caused or exacerbated by excess gastric acid secretion.

The following Examples illustrate the preparation of active compounds of the formula (I). The following Descriptions illustrate the preparation of intermediates thereto.

All temperatures are in degrees Celsius.

The following Pharmacological Data Section illustrates the useful activity of the compounds.

0167943

Description 1

4-Chloro-2,3-dimethylpyridine-1-oxide (D1)

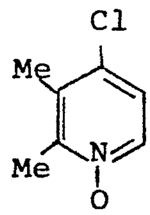

D.1

2,3-Dimethyl-4-nitropyridine-1-oxide (1.3g) was added portionwise to acetyl chloride (6ml) at 0°C. The mixture was allowed to warm to room temperature for 7 minutes before pouring onto crushed ice. The solution was made alkaline (pH 9) with potassium carbonate and extrated with chloroform (2 x 30ml). The combined organic phase was washed with brine (50ml) and dried ($MgSO_4$). Evaporation of the solvent gave a yellow oil (1.13g) which was purified by column chromatography (Silica gel - Merck 7734) eluting with chloroform - 5% methanol:95% chloroform to give 4-chloro-2,3-dimethylpyridine-1-oxide (0.94g) as a pale yellow solid (77%).

$^1H$-NMR ($CDCl_3/CCl_4$)

$\delta$ = 2.4 (s, 3H)
$\delta$ = 2.5 (s, 3H)
$\delta$ = 7.1 (d, 1H)
$\delta$ = 8.0 (d, 1H)

Description 2

(4-Benzyloxy-3-methylpyrid-2-yl)methyl acetate (D2)

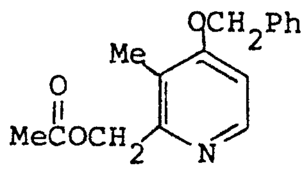

D.2

4-Chloro-2,3-dimethylpyridine-1-oxide (3,4g; 22mmol) was added to a mixture of potassium t-butoxide (2.9g, 24.2mmol) in benzylalcohol (15ml). The mixture was heated to 100°C for 72hrs followed by evaporation of most of the benzyl alcohol under high vacuum. The residue was partitioned between water (100ml) and chloroform (100ml). The organic phase was washed with brine (70ml), dried ($MgSO_4$) and evaporated in vacuo. The residue was then heated under high vacuum to remove residual benzyl alcohol.

A solution of the crude 4-benzyloxy-2,3-dimethylpyridine-1-oxide thus obtained, in acetic anhydride (23ml), was heated on a steam bath for 1 hour. Evaporation of the solvent left a brown gum which was triturated under xylene and then evaporated in vacuo. The residue was partitioned between chloroform (150ml) and dilute sodium bicarbonate solution (150ml). The organic phase was washed with brine (70ml) dried ($Na_2SO_4$) and concentrated in vacuo to give a black oil. Column chromatography (silica gel - Merck 7734), eluting with ethyl acetate, gave the (4-benzyloxy-3-methylpyrid-2-yl)methyl acetate as a pale brown oil (3.81g) (65%).

$^1$H-NMR ($CCl_4$)

δ = 2.0 (s, 3H)
δ = 2.2 (s, 3H)
δ = 5.0 (s, 2H)
δ = 5.1 (s, 2H)
δ = 6.6 (d, 1H)
δ = 7.3 (s, 5H)
δ = 8.1 (d, 1H)

0167943

Description 3

4-Benzyloxy-3-methylpyridine-2-methanol (D3)

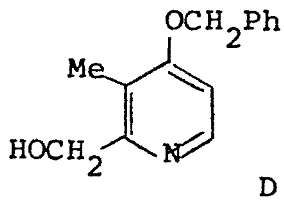

D.3

A solution of (4-benzyloxy-3-methylpyrid-2-yl)methyl acetate (3.81g, 14mmol) and 10% sodium hydroxide solution (14.1ml) in ethanol (50ml) was heated on a steam bath for 30 minutes. The solvent was evaporated *in vacuo*, and the residue partitioned between chloroform (100ml) and water (100ml). The organic phase was washed with brine (75ml), dried ($Na_2SO_4$) and evaporated *in vacuo* to give the 4-benzyloxy-3-methyl-pyridine-2-methanol (3.17g) as a yellow solid (98%).

$^1H$-NMR ($CDCl_3/CCl_4$)

$\delta$ = 2.1 (s, 3H)
$\delta$ = 4.6 (brs, 3H)
$\delta$ = 5.1 (s, 2H)
$\delta$ = 6.7 (d, 1H)
$\delta$ = 7.3 (s, 5H)
$\delta$ = 8.2 (brm, 1H)

Description 4

4-Benzyloxy-2-chloromethyl-3-methylpyridine hydrochloride (D4)

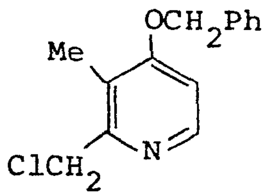

.HCl D.4

Thionyl chloride (0.6ml, 8mmol) was added to a solution of 4-benzyloxy-3-methyl-pyridine-2-methanol (460mg, 2mmol) in chloroform (10ml) at room temperature. The solution was then heated under reflux for 30 minutes. After cooling the solvent was evaporated *in vacuo*, triturated under xylene and re-evaporated *in vacuo*. The residue was triturated under ether to yield the 4-benzyloxy-2-chloromethyl-3-methylpyridine hydrochloride (0.57g) as an off-white solid (mp 160-1°C).

$^1H$-NMR ($CDCl_3$ + $d^6$-DMSO)

$\delta$ = 2.4 (s, 3H)

$\delta$ = 5.0 (s, 2H)

$\delta$ = 5.3 (s, 2H)

$\delta$ = 7.1-7.7 (m, 7H)

$\delta$ = 8.4 (d, 1H)

0167943

Example 1a

2-[(4-Benzyloxy-3-methylpyrid-2-yl)methylthio]benzimidazole (E1a)

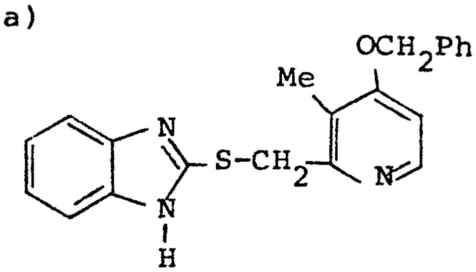

E.1a

A solution of 4-benzyloxy-2-chloromethyl-3-methylpyridine hydrochloride (0.57g, 2mmol) in ethanol (20ml) was added to a solution of 2-mercaptobenzimidazole (0.316g, 2.1mmol) and 10% sodium hyroxide solution (1.6ml, 4mmol) in ethanol (20ml). The solution was allowed to stand overnight. Solvent was evaporated in vacuo and the residue partitioned between chloroform (25ml) and water (25ml) and the aqueous phase was further extracted with chloroform (25ml). The combined organic extracts were washed with brine (35ml), dried ($Na_2SO_4$) and evaporated in vacuo to give an oily solid (0.78g). Recrystallisation from ethyl acetate gave 2-[(4-benzyloxy-3-methylpyrid-2-yl)methylthio]benzimidazole (0.58g) as a white solid (80%) (mp 189-191$^oC$).

$^1H$-NMR ($CDCl_3$-$d^4$ MeOH-$d^6$-DMSO)

$\delta$ = 2.3 (s, 3H)
$\delta$ = 4.7 (s, 2H)
$\delta$ = 5.2 (s, 2H)
$\delta$ = 6.9 (d, 1H)
$\delta$ = 7.0-7.7 (m, 9H - includes singlet at $\delta$.7.4)
$\delta$ = 8.3 (d, 1H)

Similarly prepared were:-

Example 2a:

2-[(4-benzyloxy-3-methylpyrid-2-yl)methylthio]-5-methoxy-benzimidazole (E2a);
$^{1}H$-NMR ($CDCl_3$-$d^4$MeOH) $\delta$ = 2.3(s, 3H); 3.8(s, 3H); 4.4(s, 2H); 5.1(s, 2H); 6.6-7.0(m, 3H); 7.1-7.5(m, 6H); 8.2(d, 1H).

Example 3a:

2-[(4-benzyloxy-3-methylpyrid-2-yl)methylthio]-5-bromo-6-nitro-benzimidazole.

Example 4a:

2-[(4-benzyloxy-3-methylpyrid-2-yl)methylthio]-5-trifluoromethylbenzimidazole;
$^{1}H$-NMR ($CDCl_3$-$d^4$-MeOH) $\delta$ = 2.3(s, 3H); 4.5(s, 2H); 5.1(s, 2H); 6.7(d, 1H); 7.2-7.6(m, 7H); 7.7(s, 1H); 8.2(d, 1H).

Example 5a:

2-[(4-benzyloxy-3-methylpyrid-2-yl)methylthio]-5-methyl benzimidazole;
$^{1}H$-NMR ($CDCl_3$-$d^4$-MeOH) $\delta$ = 2.3(s, 3H); 2.4(s, 3H); 4.4(s, 2H); 5.1(s, 2H); 6.6-7.1(m, 7H); 8.2(d, 1H).

Example 6a:

5-Amino-6-bromo-2-[(4-benzyloxy-3-methylpyrid-2-yl)-methylthio]-benzimidazole

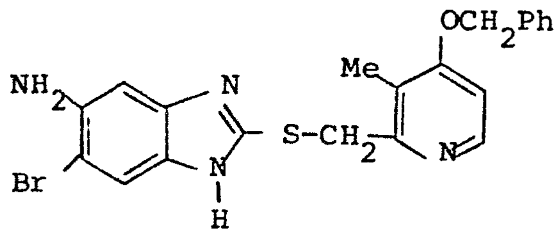

E.6a

Stannous chloride (0.51g, 2.7mmol) was added portionwise to a solution of 2-[(4-benzyloxy-3-methylpyrid-2-yl)methylthio]-5-bromo-6-nitrobenzimidazole (E3a) (0.327g, 0.7mmol) and 5N hydrochloric acid (1.2ml) in ethanol (30ml). The solution was allowed to stand overnight. The solution was made basic with saturated potassium carbonate (pH 11) and extracted with ethyl acetate (3 x 30ml). The combined organic phase was washed with water (2 x 30ml), brine (50ml) and dried ($Na_2SO_4$). Evaporation of solvent gave an oily solid. Column chromatography (silica gel, Merck 7734), eluting with chloroform - 5% methanol:95% chloroform, gave the 5-amino-6-bromo-2-[(4-benzyloxy-3-methylpyrid-2-yl)methylthio]-benzimidazole (6a) (0.21g) as a yellow solid (73%).

$^1H$-NMR ($CDCl_3$)

$\delta$ = 2.2 (s, 3H)
$\delta$ = 4.3 (s, 2H)
$\delta$ = 5.0 (s, 2H)
$\delta$ = 6.6 (d, 1H)
$\delta$ = 6.7 (s, 1H)
$\delta$ = 7.2 (s, 5H)
$\delta$ = 7.5 (s, 1H)
$\delta$ = 8.2 (d, 1H)

Example 1b

2-[(4-Benzyloxy-3-methylpyrid-2-yl)methylsulphinyl]-benzimidazole

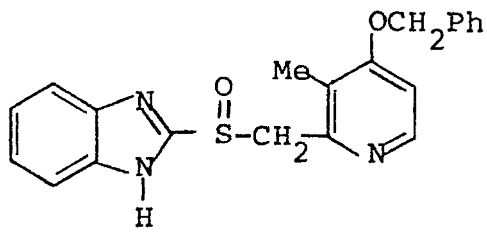

(El.b)

m-Chloroperoxybenzoic acid (131mg, 76mmol) was added to a stirred solution of 2-[(4-benzyloxy-3-methylpyrid-2-yl)methylthio]benzimidazole (Ela) (250mg, 69mmol) in dichloromethane (30ml) at 0°C under an atmosphere of nitrogen. After 5 minutes dilute sodium bicarbonate solution (30ml) was added and the mixture partitioned. The organic phase was dried ($Na_2SO_4$) and evaporated *in vacuo* to give a dark oil. Trituration under acetonitrile (2ml) gave a solid which was filtered, washed with ether and dried *in vacuo* to give the 2-[(4-benzyloxy-3-methylpyrid-2-yl)methylsulphinyl]-benzimidazole (Elb) (206mg) as a grey solid (mp 139-141°C dec).

$^1$H-NMR ($CDCl_3$)

δ = 2.22 (s, 3H)
δ = 4.83 (s, 2H)
δ = 5.09 (s, 2H)
δ = 6.27 (d, 1H)
δ = 7.15-7.8 (m, 10H, includes singlet at δ = 7.42)
δ = 8.33 (d, 1H)

Similarly were prepared:-

Example 2b:

2-[(4-benzyloxy-3 - methylpyrió-2-yl)methylsulphinyl]-5-methoxy-benzimidazole (E2b):
$^1H$-NMR ($CD_2Cl_2$) δ = 2.15(s, 3H); 3.78(s, 3H); 4.75(s, 2H); 5.06(s, 2H); 6.65-7.1 (m, 3H); 7.2-7.6(m, 6H); 8.27(d, 1H).

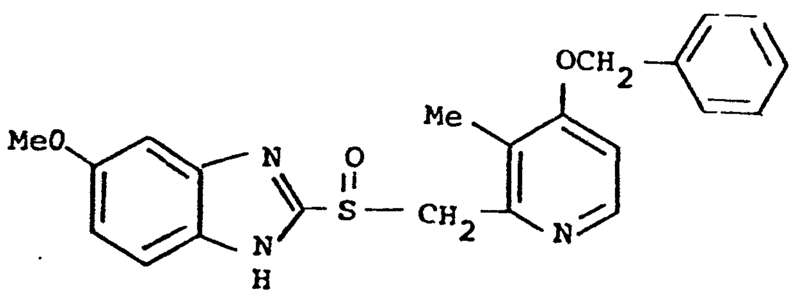

Example 7a

2-[(4-[4-Methoxybenzyloxy]-3-methylpyrid-2-yl)methyl-thio]benzimidazole

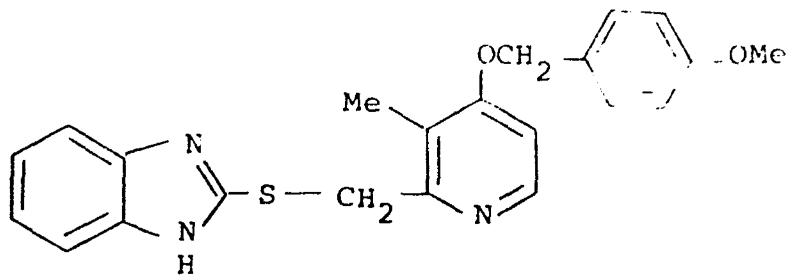

Example 8a

2-[(4-[3,5-Dichlorobenzyloxy]-3-methylpyrid-2-yl)methyl thio]benzimidazole

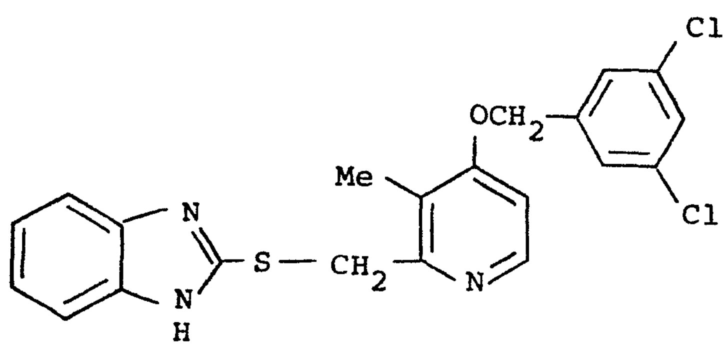

Example 9a

2-[(4-[3,5-Dichlorobenzyloxy]-3-methylpyrid-2-yl)methyl thio]-5-methylbenzimidazole

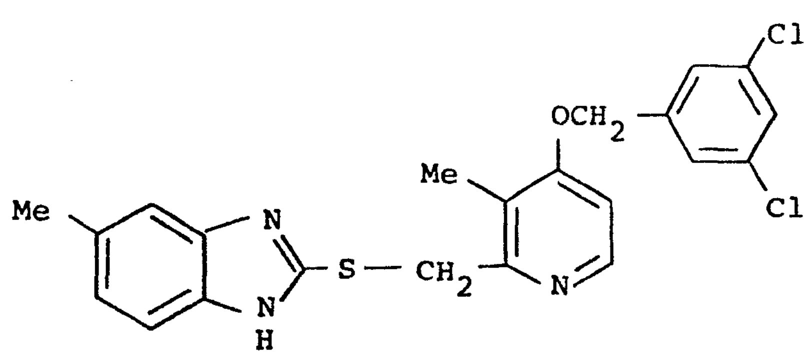

Example 10a

2-[(4-[4-Fluorobenzyloxy]-3-methylpyrid-2-yl)methylthio]benzimidazole

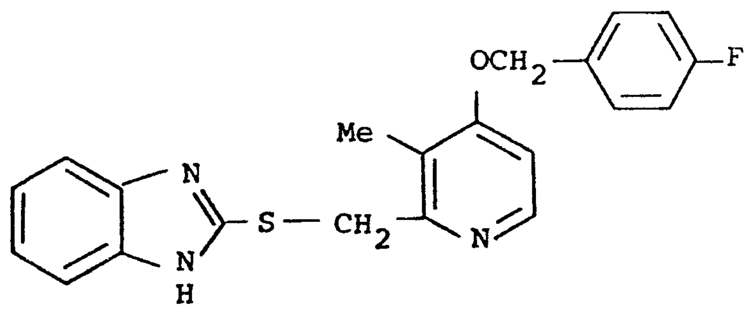

Example 11a

2-[(4-Fluorobenzyloxy]-3-methylpyrid-2-yl)methylthio]-5-methylbenzimidazole

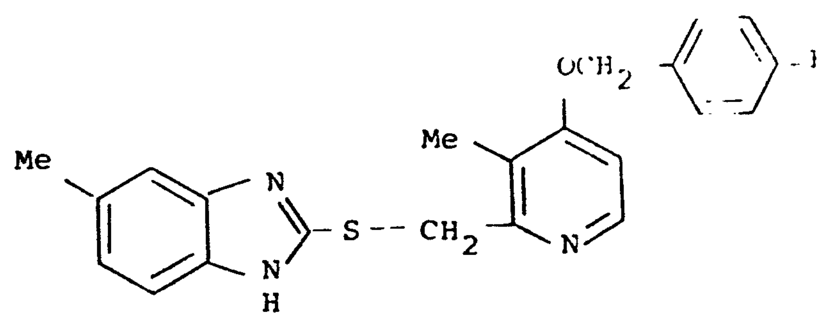

Example 12a

5-Chloro-2-[(4-[4-fluorobenzyloxy]-3-methylpyrid-2-yl)-methylthio]benzimidazole

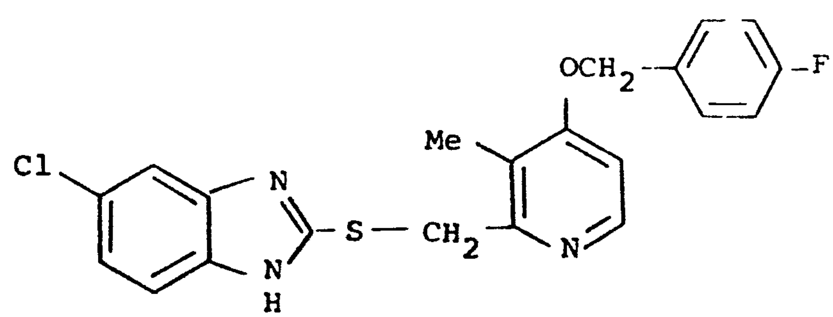

Example 13a

2-[(4-[4-Chlorobenzyloxy]-3-methylpyrid-2-yl)methylthio]benzimidazole

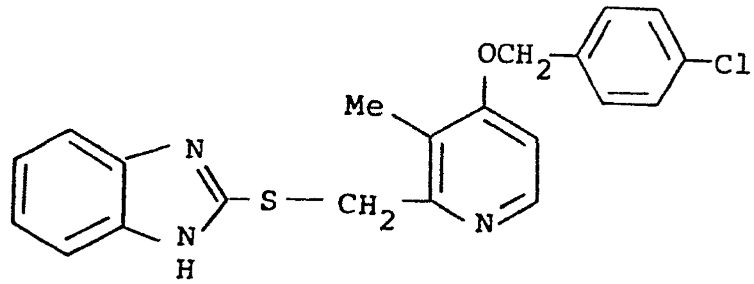

Example 14a

2-[(4-Benzyloxypyrid-2-yl)methylthio]benzimidazole

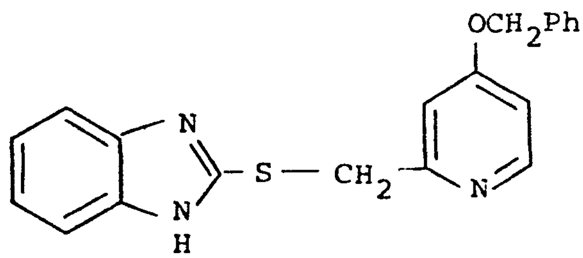

Example 15a

5-Chloro-2-[(4-[4-chlorobenzyloxy]-3-methylpyrid-2-yl)-methylthio]benzimidazole

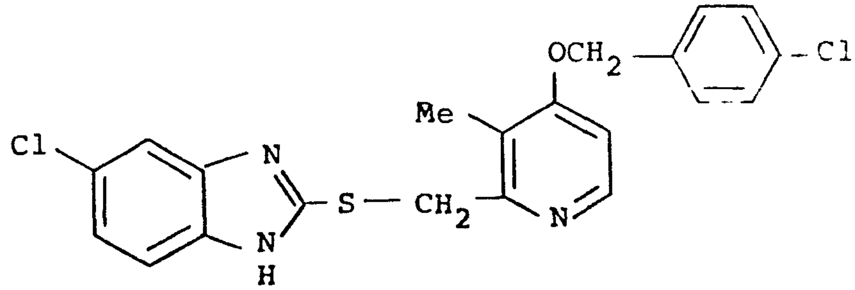

Example 16a

5-Chloro-2-[(4-[3,5-dichlorobenzyloxy]-3-methylpyrid-2-yl)methylthio]benzimidazole

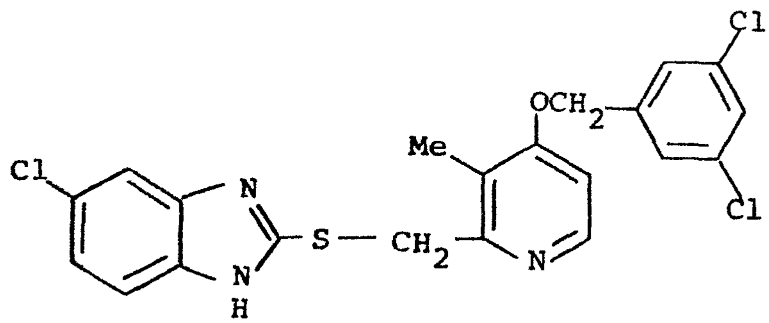

Example 17a

2-[(4-Benzyloxypyrid-2-yl)methylthio]-5-trifluoromethyl benzimidazole

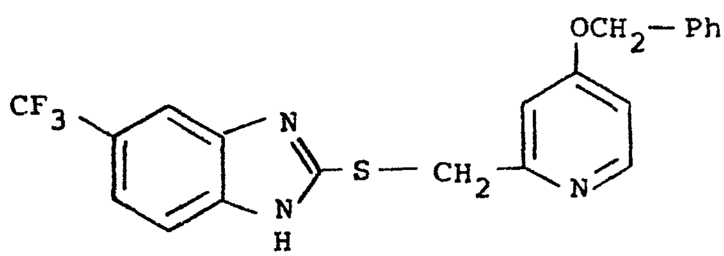

Example 18a

2-[(4'-Phenethyloxypyrid-2-yl)methylthio]benzimidazole

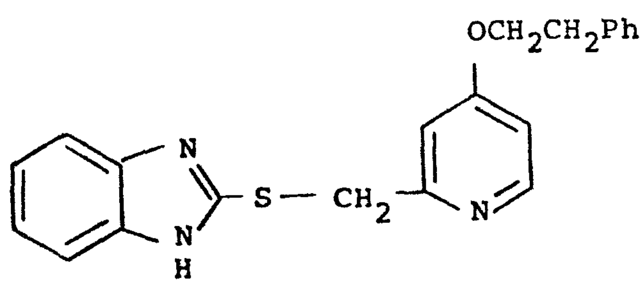

Example 19a

2-[(4-Benzyloxypyrid-2-yl)methylthio]-5-chlorobenzimidazole

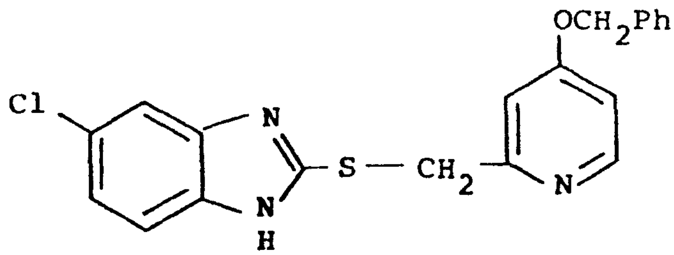

Example 20a

2-[(3-Methyl-4-phenoxypyrid-2-yl)methylthio]benzimidazole

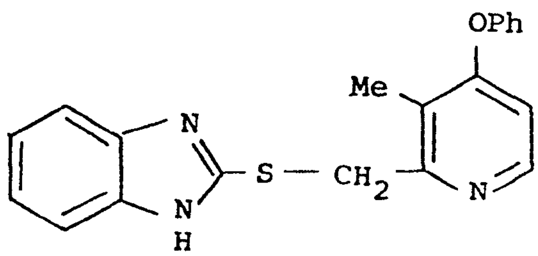

Example 21a

2-[(4-(4-Fluorobenzyloxy)pyrid-2-yl)methylthio]benzimidazole

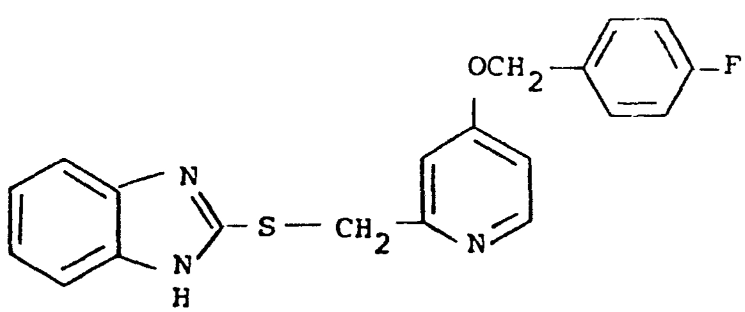

Example 22a

2-[(4-[4-Fluorophenoxy]-3-methylpyrid-2-yl)methylthio]-benzimidazole

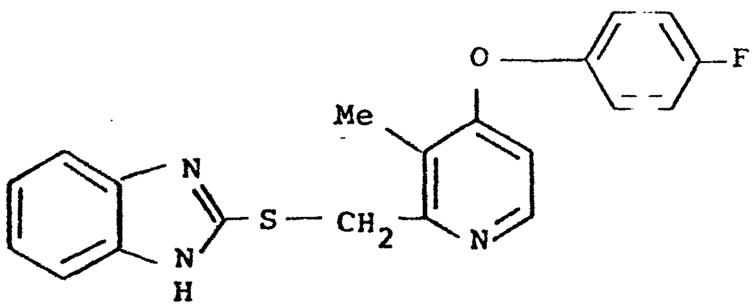

Example 23a

2-[1-([4-(4-Fluorobenzyloxy)pyrid-2-yl]ethyl)thio]-benzimidazole

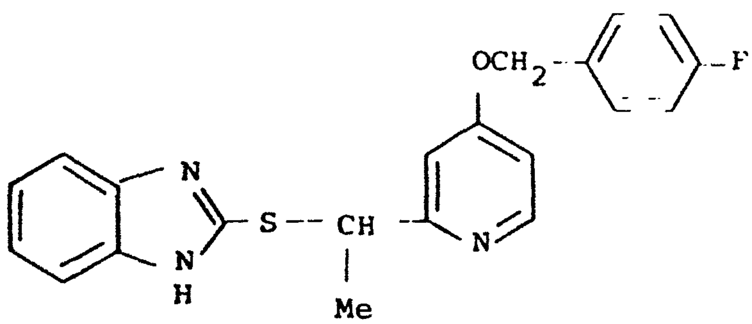

Example 24a

2-[(4-Benzyloxy-5-methylpyrid-2-yl)methylthio]benzimidazole

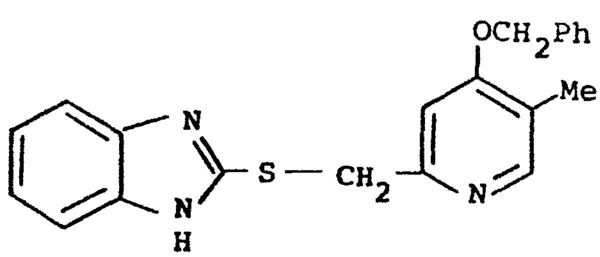

Example 25a

2-[(4-Benzyloxy-5-methylpyrid-2-yl)methylthio]-5-methoxybenzimidazole

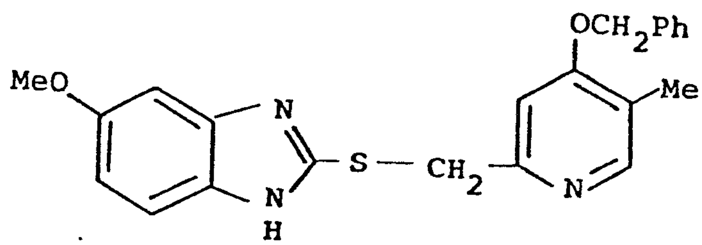

Example 26a

2-[(4-[4-Chlorobenzyloxy]-3-methylpyrid-2-yl)methylthio]-5-cyclopropylcarbonylbenzimidazole

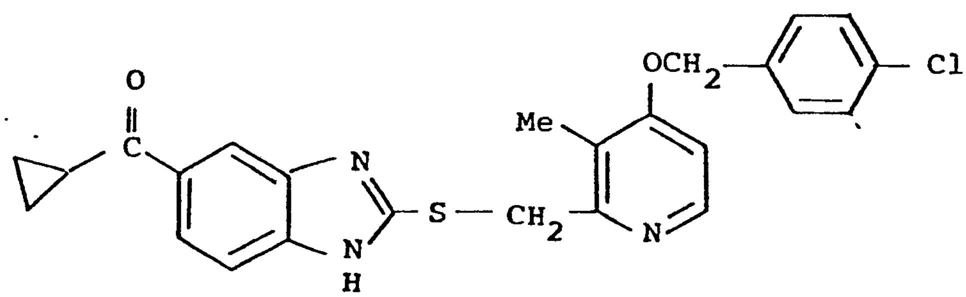

Example 27a

2-[(4-Benzyloxy-5-methylpyrid-2-yl)methylthio]-5-chloro benzimidazole

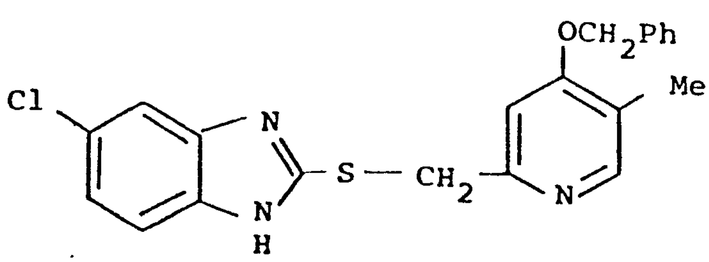

Example 28a

2-[(4-Benzyloxy-5-methylpyrid-2-yl)methylthio]-5-trifluoromethylbenzimidazole

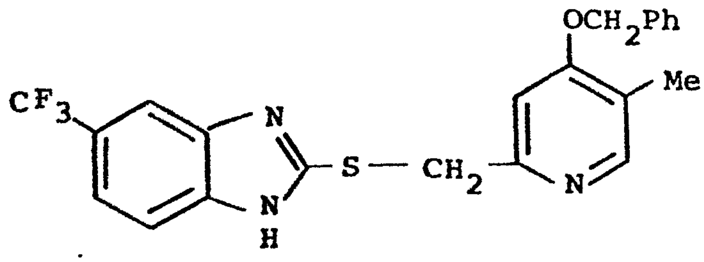

Example 29a

2-[(4-[4-Fluorobenzyloxy]-5-methylpyrid-2-yl)methylthio]benzimidazole

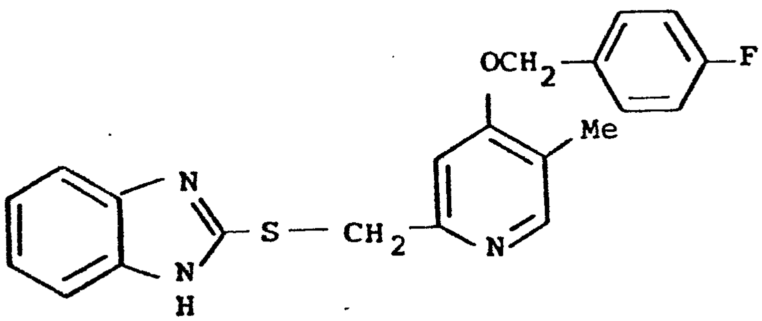

Example 30a

2-[(4-[4-Fluorobenzyloxy]-5-methylpyrid-2-yl)methylthio]-5-methylbenzimidazole

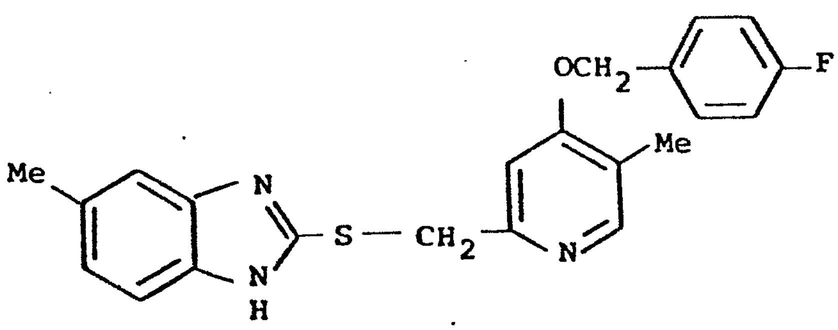

Example 31a

2-[(4-Benzyloxy-5-methylpyrid-2-yl)methylthio]-5-methyl benzimidazole

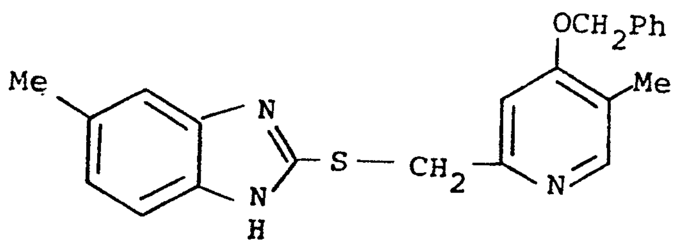

Example 4b

2-[(4-Benzyloxy-3-methylpyrid-2-yl)methylsulphinyl-5-trifluoromethylbenzimidazole

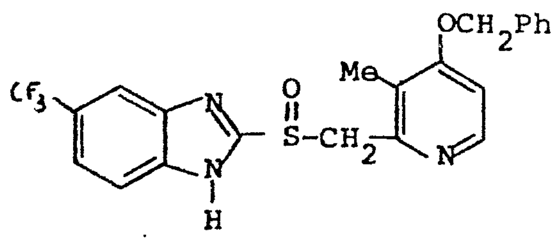

$^1$H-NMR ($CD_2Cl_2$)

δ 2.17 (s, 3H)
4.70 (d, 1H)
4.71 (d, 1H)
5.06 (s, 2H)
6.79 (d, 1H)
7.38 (s, 5H)
7.51 (dd, 1H)
7.69 (d, 1H)
7.92 (s, 1H)
8.25 (d, 1H)

Example 5b

2-[(4-Benzyloxy-3-methylpyrid-2-yl)methylsulphinyl-5-methylbenzimidazole

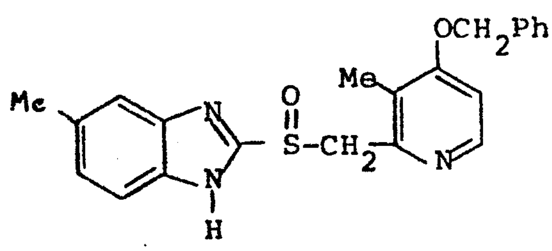

$^1H$-NMR ($CD_2Cl_2$)

δ 2.14 (s, 3H)
2.42 (s, 3H)
4.57 (d, 1H)
4.93 (d, 1H)
5.05 (s, 2H)
6.79 (d, 1H)
6.99-7.69 (m, 8H, includes singlet at δ = 7.38)
8.30 (d, 1H)

Example 6b

5-Amino-6-bromo-2-[(4-benzyloxy-3-methylpyrid-2-yl)-methylsulphinyl]-benzimidazole

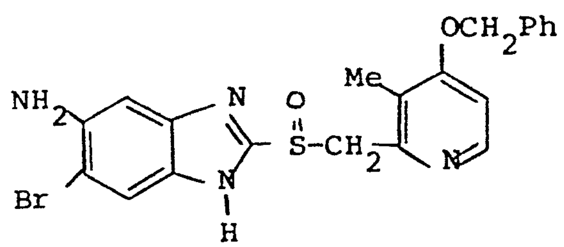

$^1H$-NMR ($CD_2Cl_2$ + $d^6$-DMSO)

δ 2.19 (s, 3H)
4.68 (s, 2H)
4.90 (br.s, 2H)
5.17 (s, 2H)
6.92 (d, 1H)
7.05 (s, 1H)
7.28-7.55 (m, 5H)
7.75 (s, 1H)
8.27 (d, 1H)
12.95 (br.s, 1H)

Example 7b

2-[(4-[4-methoxybenzyloxy]-3-methylpyrid-2-yl)methyl-sulphinyl]benzimidazole

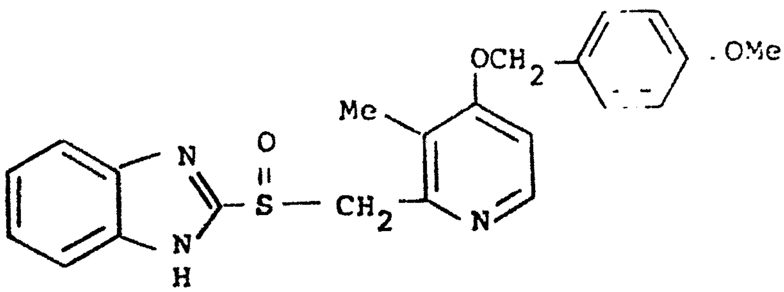

$^1H$-NMR ($CD_2Cl_2$)

| | | |
|---|---|---|
| δ | 2.14 | (s, 3H) |
| | 3.80 | (s, 3H) |
| | 4.67 | (d, 1H) |
| | 4.87 | (d, 1H) |
| | 5.01 | (s, 2H) |
| | 6.74-7.04 | (m, 3H) |
| | 7.16-7.48 | (m, 4H) |
| | 7.48-7.73 | (m, 2H) |
| | 8.31 | (d, 1H) |

Example 8b

2-[(4-[3,5-dichlorobenzyloxy]-3-methylpyrid-2-yl)methyl sulphinyl]benzimidazole

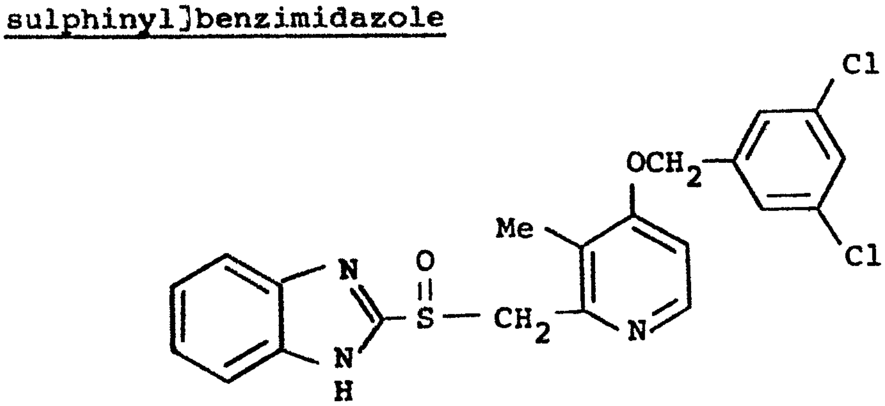

Mass spectrum $C_{21}H_{15}N_3OSCl_2$ required 427.0313
observed 427.0319

Example 9b

2-[(4-[3,5-dichlorobenzyloxy]-3-methylpyrid-2-yl)methyl sulphinyl]-5-methylbenzimidazole

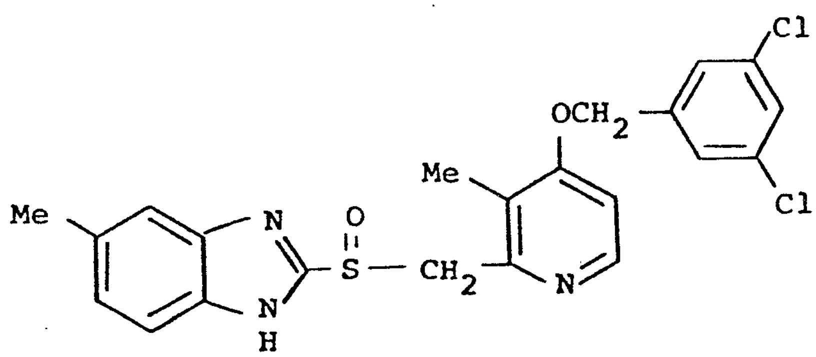

$^1$H-NMR ($CD_2Cl_2$)

2.19 (s, 3H)
2.45 (s, 3H)
4.60 (d, 1H)
4.85 (d, 1H)
5.03 (s, 2H)
6.75 (d, 1H)
7.00-7.62 (m, 6H)
8.32 (d, 1H)

Example 10b

2-[(4-[4-Fluorobenzyloxy]-3-methylpyrid-2-yl)methyl-sulphinyl]benzimidazole

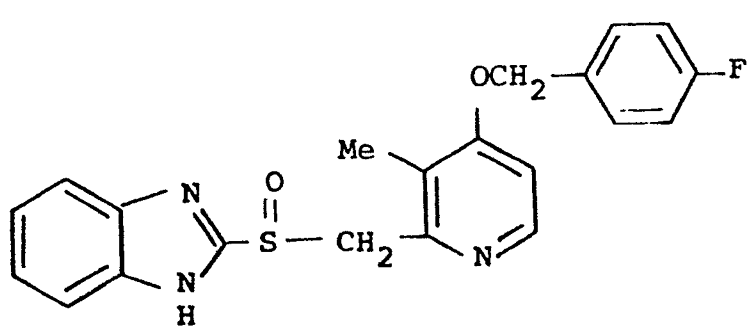

$^1H$-NMR ($d^6DMSO$)

| | | |
|---|---|---|
| δ | 2.18 | (s, 3H) |
| | 4.68 | (d, 1H) |
| | 4.87 | (d, 1H) |
| | 5.21 | (s, 2H) |
| | 7.00-7.80 | (m, 9H) |
| | 8.27 | (d, 1H) |

Example 11b

2-[(4-[4-Fluorobenzyloxy]-3-methylpyrid-2-yl)methylsulphinyl]-5-methylbenzimidazole

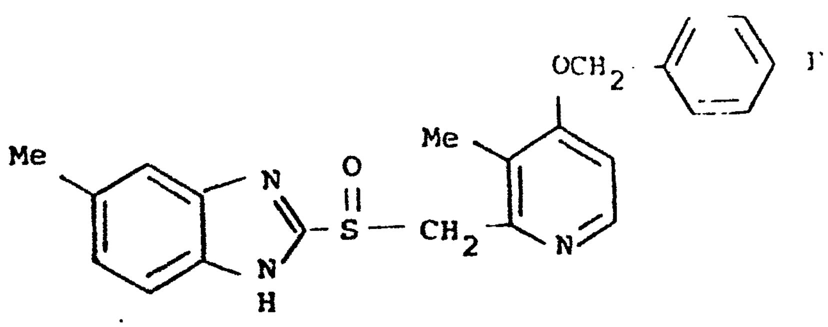

$^1H$-NMR ($CD_2Cl_2$)

| | | |
|---|---|---|
| δ | 2.14 | (s, 3H) |
| | 2.44 | (s, 3H) |
| | 4.66 | (d, 1H) |
| | 4.85 | (d, 1H) |
| | 5.03 | (s, 2H) |
| | 6.79 | (d, 1H) |
| | 6.91-7.65 | (m, 8H) |
| | 8.30 | (d, 1H) |

Example 12b

5-Chloro-2-[(4-[4-Fluorobenzyloxy]-3-methylpyrid-2-yl)-methylsulphinyl]benzimidazole

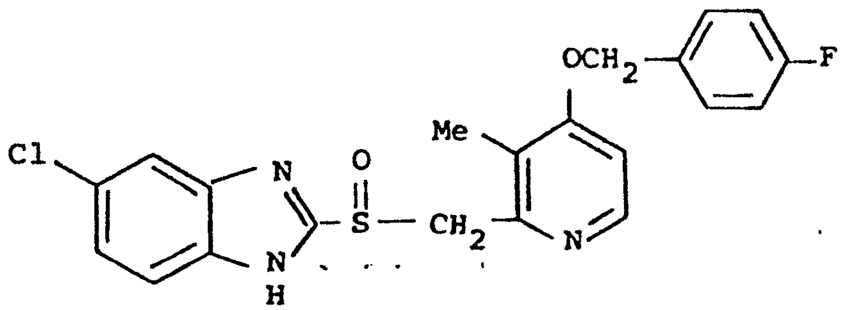

$^1$H-NMR ($CD_2Cl_2$ + $d^6$DMSO)

| | | |
|---|---|---|
| δ | 2.21 | (s, 3H) |
| | 4.72 | (s, 2H) |
| | 5.14 | (s, 2H) |
| | 6.89 | (d, 1H) |
| | 6.78-7.71 | (m, 7H) |
| | 8.28 | (d, 1H) |

Example 13b

2-[(4-[4-Chlorobenzyloxy]-3-methylpyrid-2-yl)methyl-sulphinyl]benzimidazole

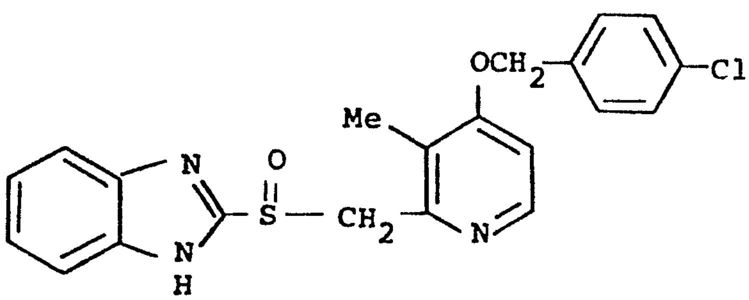

$^1$H-NMR ($CD_2Cl_2$)

δ 2.19 (s, 3H)
4.47 (d, 1H)
4.85 (d, 1H)
5.08 (s, 2H)
6.81 (d, 1H)
7.16-7.48 (m, 6H, includes singlet at δ = 7.38)
7.48-7.80 (m, 2H)
8.31 (d, 1H)

Example 14b

2-[(4-Benzyloxypyrid-2yl)methylsulphinyl]benzimidazole

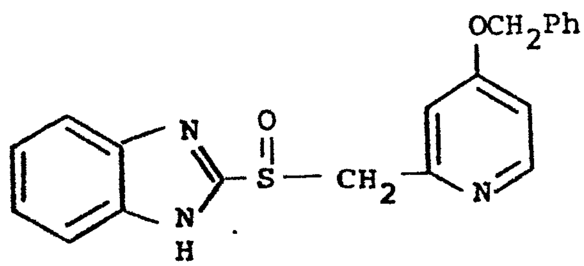

$^1$H-NMR ($CD_2Cl_2$ + $d^6$-DMSO)

δ 4.47 (d, 1H)
4.72 (d, 1H)
4.85 (s, 2H)
6.70 (d, 1H)
6.83 (dd, 1H)
7.16-7.51 (m, 7H, includes singlet at δ = 7.37)
7.51-7.84 (m, 2H)
8.37 (d, 1H)
12.95 (br.s, 1H)

Example 15b

5-Chloro-2-[(4-[4-chlorobenzyloxy]-3-methylpyrid-2-yl)-methylsulphinyl]benzimidazole

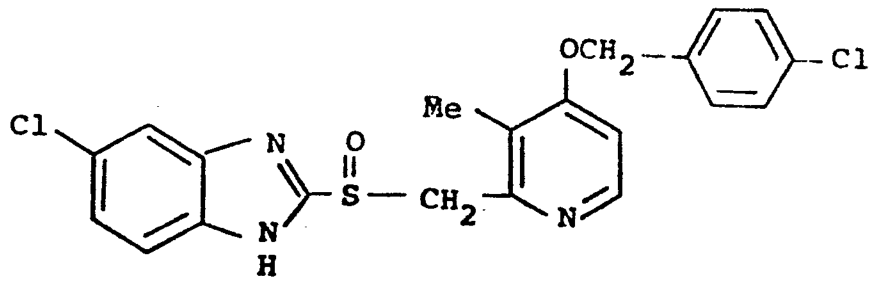

$^1$H-NMR ($CD_2Cl_2$)

δ 2.20 (s, 3H)
4.60 (d, 1H)
4.85 (d, 1H)
5.10 (s, 2H)
6.80 (d, 1H)
7.29 (dd, 1H)
7.40 (s, 4H)
7.59 (d, 1H)
7.63 (s, 1H)
8.31 (d, 1H)

Example 16b

5-Chloro-2-[(4-[3,5-dichlorobenzyloxy]-3-methylpyrid-2-yl)methylsulphinyl]benzimidazole

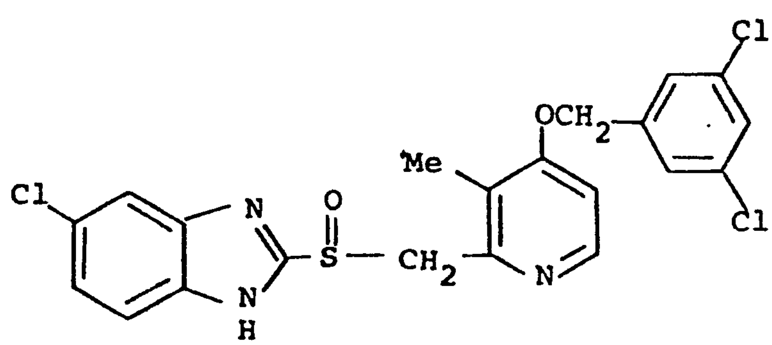

$^1H$-NMR ($CD_2Cl_2$ + $d^6$-DMSO)

δ 2.22 (s, 3H)
4.63 (d, 1H)
4.80 (d, 1H)
5.13 (s, 2H)
6.86 (d, 1H)
7.27 (dd, 1H)
7.41 (s, 3H)
7.61 (d, 1H)
7.68 (s, 1H)
8.27 (d, 1H)

Example 17b

2-[(4-Benzyloxypyrid-2-yl)methylsulphinyl]-5-trifluoromethylbenzimidazole

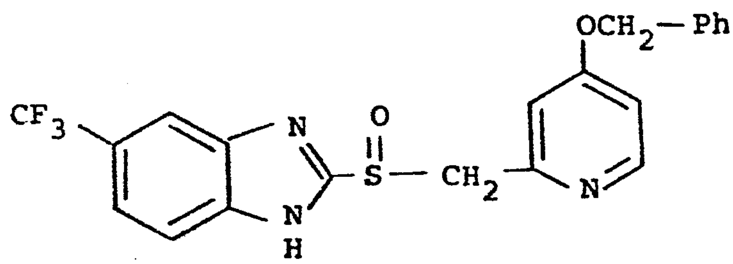

$^1H$-NMR ($CD_2Cl_2$)

δ 4.45-4.93 (m, 4H)
6.61-6.87 (m, 2H)
7.11-7.46 (m, 5H)
7.56 (dd, 1H)
7.76 (d, 1H)
7.98 (s, 1H)
8.29 (d, 1H)
9.62 (br.s, 1H)

Example 18b

2-[(4'-Phenethyloxypyrid-2-yl)methylsulphinyl]benzimidazole

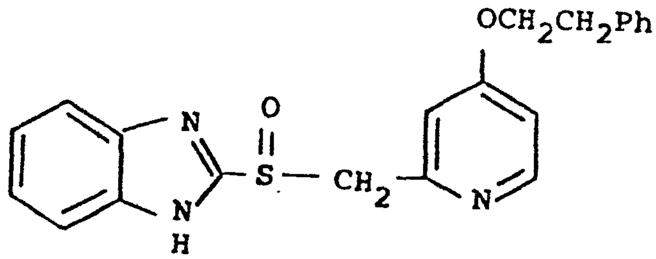

$^1H$-NMR ($CD_2Cl_2$)

| | |
|---|---|
| δ 2.91 | (t, 2H) |
| 3.77-4.01 | (m, 2H) |
| 4.48 | (d, 1H) |
| 4.73 | (d, 1H) |
| 6.54-6.77 | (m, 2H) |
| 7.09-7.50 | (m, 7H) |
| 7.50-7.75 | (m, 2H) |
| 8.29 | (d, 1H) |

Example 19b

2-[(4-Benzyloxypyrid-2-yl)methylsulphinyl]-5-chlorobenzimidazole

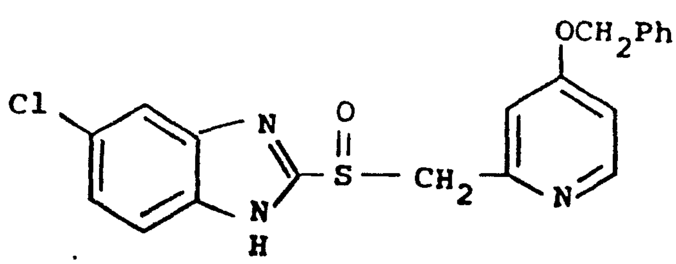

$^1H$-NMR ($CD_2Cl_2$ + $d^6$-DMSO)

δ 4.45 (d, 1H)
4.70 (d, 1H)
4.88 (s, 2H)
6.66-6.90 (m, 2H)
7.18-7.68 (m, 8H, includes singlet at δ = 7.29)
8.34 (d, 1H)

Example 20b

2-[(3-Methyl-4-phenoxypyrid-2-yl)methylsulphinyl]-benzimidazole

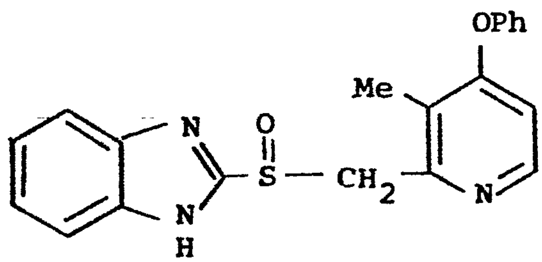

$^1H$-NMR ($CD_2Cl_2$)

δ 2.26 (s, 3H)
4.72 (d, 1H)
4.92 (d, 1H)
6.55 (d, 1H)
6.83-7.09 (m, 2H)
7.09-7.46 (m, 5H)
7.46-7.87 (m, 2H)

Example 21b

2-[(4-(4-Fluorobenzyloxy)pyrid-2-yl)methylsulphinyl]-benzimidazole

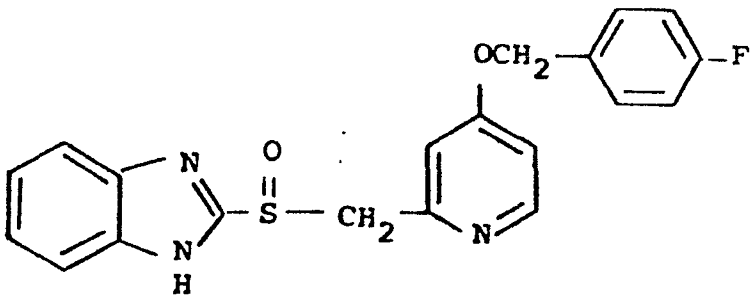

$^1$H-NMR ($CD_2Cl_2$)

| | | |
|---|---|---|
| δ | 4.50-4.77 | (m, 4H) |
| | 7.60 | (d, 1H) |
| | 7.74 | (dd, 1H) |
| | 6.98-7.09 | (m, 2H) |
| | 7.16-7.26 | (m, 2H) |
| | 7.26-7.35 | (m, 2H) |
| | 7.40-7.90 | (m, 2H) |
| | 8.25 | (d, 1H) |
| | 12.26 | (br.s, 1H) |

Example 22b

2-(4-[4-Fluorophenoxy]-3-methylpyrid-2-yl)methylsulphinyl]benzimidazole

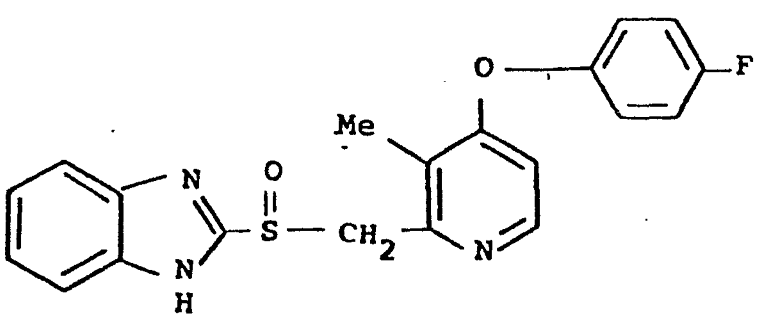

$^1H$-NMR ($CD_2Cl_2$)

δ 2.25 (s, 3H)
4.71 (d, 1H)
4.91 (d, 1H)
6.52 (d, 1H)
6.82-7.45 (m, 6H)
7.45-7.84 (m, 2H)
8.24 (d, 1H)
12.3 (br.s, 1H)

Example 23b

2-[1-([4-(4-Fluorobenzyloxy)pyrid-2-yl]ethyl)sulphinyl] benzimidazole

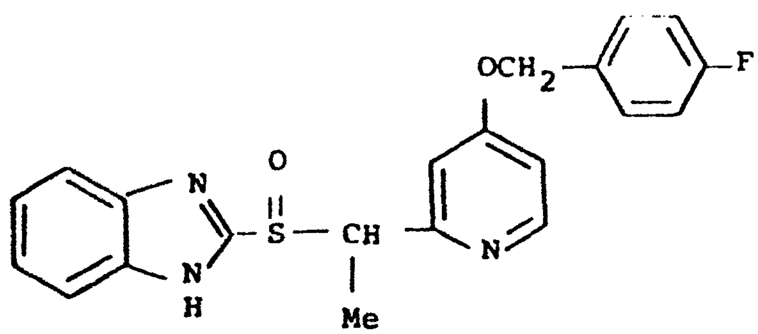

$^1H$-NMR ($CD_2Cl_2$)

δ 1.80 (d, 3H)
4.20-4.78 (m, 3H)
6.47 (d, 1H)
6.65 (d,d, 1H)
6.73-7.94 (m, 8H)
8.23 (d, 1H)
12.05 (br.s, 1H)

Example 24b

2-[(4-Benzyloxy-5-methylpyrid-2-yl)methylsulphinyl]-benzimidazole

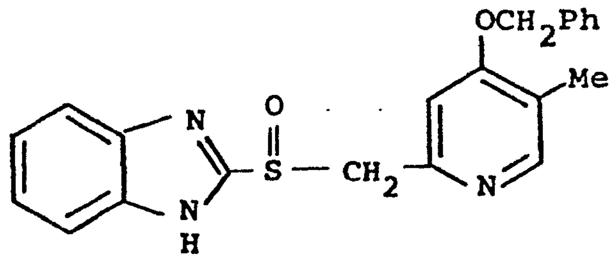

$^1$H-NMR ($CD_2Cl_2$)

| | | |
|---|---|---|
| δ | 2.11 | (s, 3H) |
| | 4.34-4.84 | (m, 4H) |
| | 6.50 | (s, 1H) |
| | 7.13-7.47 | (m, 7H) |
| | 7.47-7.77 | (m, 2H) |
| | 8.15 | (s, 1H) |

Example 25b

2-[(4-Benzyloxy-5-methylpyrid-2-yl)methylsulphinyl]-5-methoxybenzimidazole

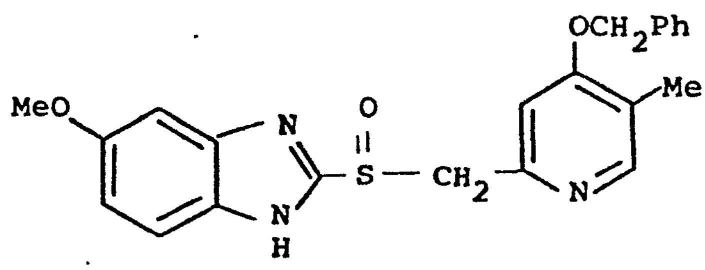

$^1$H-NMR ($CD_2Cl_2$)

δ 2.10 (s, 3H)
3.80 (s, 3H)
4.32-4.82 (m, 4H)
6.48 (s, 1H)
6.83-7.12 (m, 2H)
7.12-7.65 (m, 6H)
8.15 (s, 1H)

Example 26b

2-[(4-[4-Chlorobenzyloxy]-3-methylpyrid-2-yl)methyl-sulphinyl]-5-cyclopropylcarbonylbenzimidazole

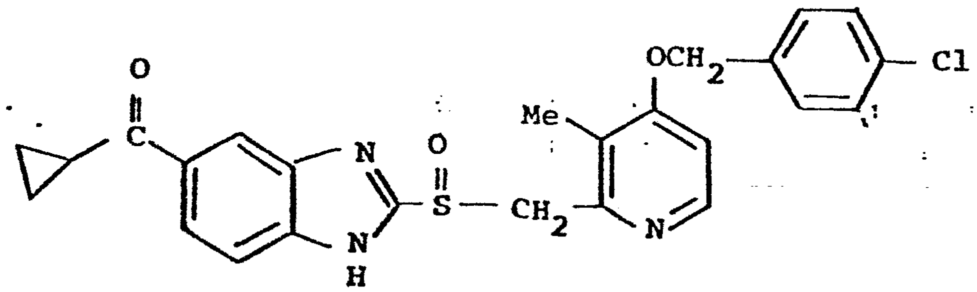

$^1$H-NMR ($CDCl_3$)

δ 8.35 (s, 1H)
8.30 (d, 1H)
8.04 (dd, 1H)
7.64 (d, 1H)
7.50-7.20 (m, 4H)
6.75 (d, 1H)
5.03 (s, 2H)
4.82 (s, 2H)
2.90-2.50 (m, 1H)
2.20 (s, 3H)
1.35-0.90 (m, 4H)

Example 27b

2-[(4-Benzyloxy-5-methylpyrid-2-yl)methylsulphinyl]-5-chlorobenzimidazole

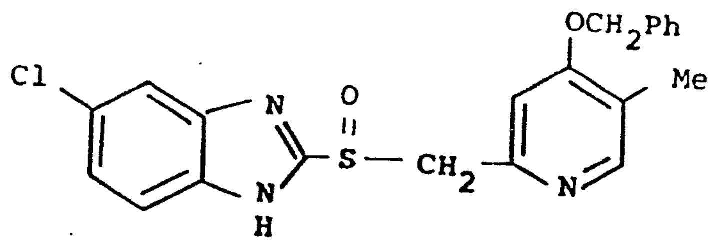

$^1$H-NMR ($CD_2Cl_2$)

δ 8.12 (s, 1H)
7.66 (s, 1H)
7.61 (d, 1H)
7.50-7.10 (m, 6H)
6.55 (s, 1H)
4.90-4.40 (m, 4H)
2.12 (s, 3H)

Example 28b

2-[(4-Benzyloxy-5-methylpyrid-2-yl)methylsulphinyl]-5-trifluoromethylbenzimidazole

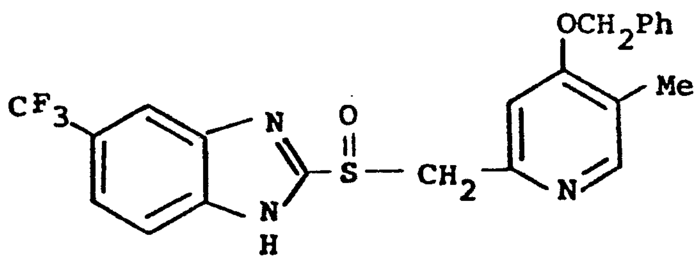

$^1H$-NMR ($d^6$-DMSO)

δ 8.20 (s, 1H)
8.01 (br.s, 1H)
7.83 (d, 1H)
7.57 (dd, 1H)
7.36 (s, 5H)
6.67 (s, 1H)
4.90-4.40 (m, 4H)
2.18 (s, 3H)

Example 29b

2-[(4-[4-Fluorobenzyloxy]-5-methylpyrid-2-yl)methylsulphinyl]benzimidazole

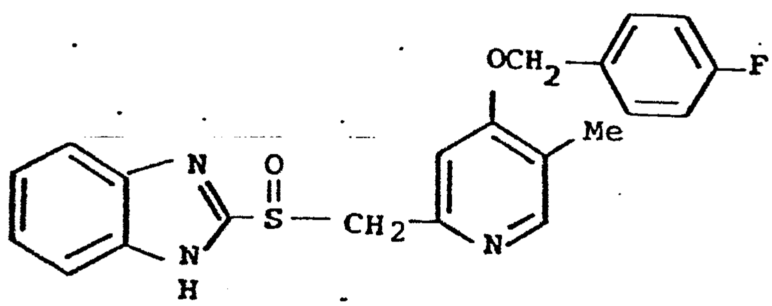

$^1H$-NMR ($CD_2Cl_2$ + $d^6$-DMSO)

δ 8.21 (s, 1H)
7.80-7.55 (m, 2H)
7.45-6.90 (m, 6H)
6.45 (s, 1H)
4.80-4.30 (m, 4H)
2.15 (s, 3H)

Example 30b

2-[(4-[4-Fluorobenzyloxy]-5-methylpyrid-2-yl)methyl-sulphinyl]-5-methylbenzimidazole

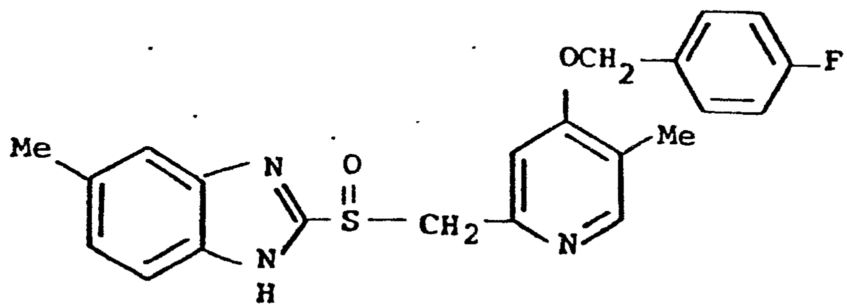

$^{1}H$-NMR ($CD_2Cl_2$)

δ 8.12 (s, 1H)
7.70-6.90 (m, 7H)
6.40 (s, 1H)
4.80-4.20 (m, 4H)
2.44 (s, 3H)
2.10 (s, 3H)

Example 31b

2-[(4-Benzyloxy-5-methylpyrid-2-yl)methylsulphinyl]-5-methylbenzimidazole

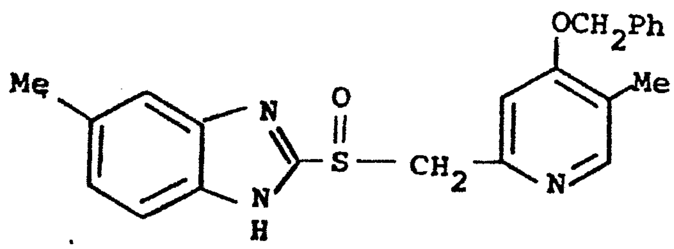

$^1$H-NMR ($CD_2Cl_2$

δ 8.13 (s, 1H)
7.60-7.00 (m, 8H)
6.45 (s, 1H)
4.80-4.25 (m, 4H)
2.44 (s, 3H)
2.10 (s, 3H)

## PHARMACOLOGY

The ability of the compounds of the invention to modify the pH of gastric acid secretion was investigated as follows:

### The perfused rat stomach preparation

The modified (1) perfused stomach preparation (2) of the urethane (25% solution) anaesthetised rat, maintained at 37°C, allows the continuous measurement of pH during basal and stimulated acid secretion.

The lumen of the stomach of male Wistar rats (approximately 200 g bodyweight) was perfused, *via* a cannula designed to reduce the dead space of the stomach, with 5% glucose solution (37°C) at the rate of 3 ml/min. The perfusate was forced over the surface of the secretory mucosa only, the antrum being excluded. The effluent then passed over a microflow type glass pH electrode *via* collecting funnel situated in the non-glandular forestomach.

The secretagogue histamine was administered as a constant intravenous infusion to produce a steady rate of acid secretion. Test compounds were administered in solution as bolus intravenous injections and any effect on the pH of the perfusate noted. The perfusate pH was recorded on a potentiometric recorder and anti-secretory responses were measured in terms of the maximal reduction in hydrogen-ion concentration expressed as a percentage of the 'control' concentrations.

## Results

Perfused rat preparation:

| Compounds | dose | Inhibition |
|---|---|---|
| 1.b | 0.5 µmol/kg | 82% |
| 7.b | 2 | 86% |
| 8.b | 2 | 80% |
| 10.b | 1 | 80% |
| 18.b | 2 | 100% |
| 20.b | 2 | 72% |
| 21.b | 2 | 100% |
| 24.b | 2.5 | 91% |

## References

1. Parsons, M.E. (1970).
Ph. D. Thesis, University of London.

2. Ghosh, M.N. and Schild, H.O. (1958).
Br. J. Pharmacol., 13, 54-61.

C

Claims

1. A compound of formula (I):

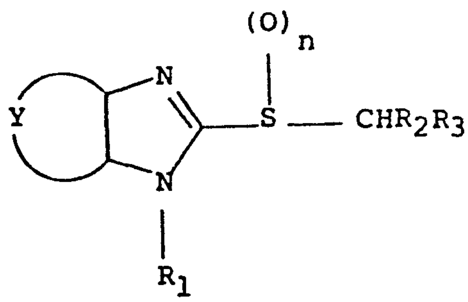

(I)

or a pharmaceutically acceptable salt, a quaternised derivative or a pharmaceutically acceptable solvent thereof;

wherein:

Y forms an optionally substituted phenyl ring;

n is zero or one;

$R_1$ is H, $C_{1-6}$ alkanoyl, $C_{1-6}$ alkanesulphonyl, or optionally substituted arylsulphonyl, aryl $C_{1-6}$ alkanoyl or aryl $C_{1-4}$ alkyl;

$R_2$ is hydrogen or $C_{1-4}$ alkyl; and

$R_3$ is pyridyl group substituted by at least one group selected from $OR_4$ or $O(CH_2)_mOR_4$ wherein $R_4$ is an optionally substituted aryl or aralkyl group of up to

10 carbon atoms and m is an integer of from 1 to 4; and by up to three further substituents one of which may be joined to $R_2$ to form a carbocylic ring of up to 7 ring atoms.

2. A compound according to claim 1 wherein $R_3$ is 2-pyridyl.

3. A compound according to claim 1 or 2 wherein $R_3$ is further substituted by one or two of halogen$C_{1-6}$ alkoxy, carboxy esterified carboxy, or amino optionally N-substituted by one or two groups independently selected from $C_{1-6}$ alkyl, phenyl or phenyl $C_{1-4}$ alkyl or optionally N.N-disubstituted by $C_{4-5}$ polymethylene or $C_{3-4}$ polymethylenecarbonyl.

4. A compound according to any one of claims 1 to 3 wherein Y is unsubstituted or includes one or two substituents selected from halo, $C_{1-6}$ alkyl, halo substituted $C_{1-6}$ alkyl, $C_{1-6}$ alkoxy, $C_{1-6}$ alkylthio, $C_{1-10}$ carboxylic acyl, $C_{1-7}$ carboxylic acylamino, carboxy, $C_{1-6}$ alkoxycarbonyl, $C_{1-6}$ alkylsulphonylamino, N-($C_{1-6}$ alkylsulphonyl)-$C_{1-4}$ alkylamino, cyano, nitro, or amino, amido or sulphonylamino any of which is optionally N-substituted by one or two groups selected from $C_{1-6}$ alkyl or phenyl or optionally N-disubstituted by $C_{4-5}$ polymethylene; or phenyl optionally substituted by one or two substituents independently selected from halo, $C_{1-6}$ alkyl, $C_{1-6}$ alkoxy, $C_{1-6}$ alkylthio, $C_{1-7}$ carboxylic acyl, $C_{1-7}$ carboxylic acylamino, $C_{1-6}$ alkylsulphonylamino, N-($C_{1-6}$ alkylsulphonyl)-$C_{1-4}$ alkylamino, cyano, or nitro, amino optionally N-substituted by one or two groups selected from $C_{1-6}$ alkyl or phenyl or optionally N-disubstituted by $C_{4-5}$ polymethylene, or carboxy or $C_{1-6}$ alkoxycarbonyl.

5. A compound according to claim 1 of formula (II):

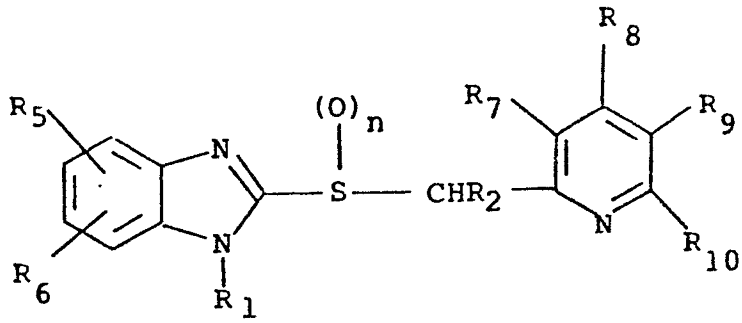

(II)

or a pharmaceutically acceptable salt, a quaternised derivative or a pharmaceutically acceptable solvate thereof;

wherein $R_1$, $R_2$ and n are as hereinbefore defined in claim 1;

$R_5$ and $R_6$ are independently hydrogen or a group selected from the substituents for Y defined in claim 1;

$R_8$ is a group selected from $OR_4$ or $-O(CH_2)m-O-R_4$;
$R_9$ and $R_{10}$ are independently selected from hydrogen, $-OR_4$,$-O(CH_2)_mR_4$, halogen, $C_{1-6}$ alkyl, $C_{1-6}$ alkoxy, $C_{1-6}$ alkylthio, $C_{1-6}$ alkoxy $C_{1-6}$ alkoxy, carboxy, esterified carboxy, or amino optionally substituted by one or two groups independently selected from $C_{1-6}$alkyl-phenyl, or phenyl-$C_{1-4}$ alkyl or optionally N,N-disubstituted by $C_{4-5}$ polymethylene or $C_{3-4}$ polymethylenecarbonyl; and $R_7$ is a group defined above for $R_{10}$ and $R_9$ or together with $R_2$ form $C_{2-9}$ alkylene; wherein $R_4$ and m are as defined in claim 1.

6. A compound according to claim 5 wherein $R_5$ and $R_6$ are independently hydrogen, chloro, bromo, methyl, trifluoromethyl, amino or methoxy.

7. A compound according to claim 5 or 6 wherein $R_1$ is hydrogen.

8. A compound according to any one of claims 5,6 or 7 wherein $R_2$ is hydrogen.

9. A compound according to any one of claims 5 to 8 wherein $R_7$, $R_9$ and $R_{10}$ are selected from hydrogen and $C_{1-4}$ alkyl.

10. A compound according to any one of claims 1 to 9 wherein $R_4$ is phenyl $C_{1-4}$ alkyl optionally substituted in the phenyl ring by one or two of halo, $C_{1-6}$ alkyl, $C_{1-6}$ alkoxy, $C_{1-6}$ alkylthio, cyano or nitro.

11. 2-[(4-Fluorobenzyloxy]-3-methylpyrid-2-yl) methylthio]benzimidazole or 2-[(4-fluorobenzyloxy]-3-methylpyrid-2-yl)methylsulphinyl]benzimidazole.

12. A process for the preparation of a compound according to any one of claims 1 to 11 or a pharmaceutically acceptable salt, a quaternised derivative or a pharmaceutically acceptable solvate thereof, which process comprises reacting a compound of formula (III) or an acid addition salt thereof:

$$R_3 - R_{11} \qquad \text{(III)}$$

wherein $R_3$ is as defined in claim 1, with a compound of formula (IV):

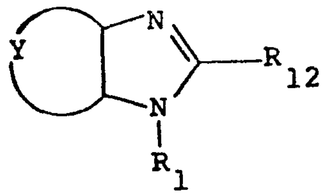

(IV)

wherein $R_1$ and Y are as defined in claim 1; and

a) $R_{11}$ is a group displaceable by a nucleophile; and
$R_{12}$ is $CH_3SO$;

b) $R_{11}$ is $CHR_2Q_1$ where $R_2$ is as defined in claim 1, and $Q_1$ is a group displaceable by a nucleophile; and
$R_{12}$ is HS; or

c) $R_{11}$ is $CHR_2SH$ where $R_2$ is as defined in claim 1; and
$R_{12}$ is a group displaceable by a nucleophile;

thereafter if desired carrying out one or more of the following steps;

(i) oxidising a compound of formula (I) wherein n is zero to a compound of formula (I) wherein n is one;
(ii) converting any variable group to another corresponding variable R group; and

(iii) salifying or quaternising the resulting compound of the formula (I).

13. A pharmaceutical composition comprising a compound according to any one of claims 1 to 11, a pharmaceutically acceptable salt, quaternised derivative or a pharmaceutically acceptable solvate thereof, together with a pharmaceutically acceptable carrier.

14. A compound according to any one of claims 1 to 11 or a pharmaceutically acceptable salt, quaternised derivative or a pharmaceutically acceptable solvate for use in the treatment or prophylaxis of disorders caused or exacerbated by excess gastric acid secretion.